# EUROPEAN PATENT APPLICATION

(11) **EP 4 682 239 A1**
(43) Date of publication of application: **21.01.2026**
(21) Application number: 23939422.4
(22) Date of filing: 28.12.2023
(51) Int. Cl.: C12M 1/34, G01N 15/14, G01N 33/48, G01N 21/01, C12N 5/00

(54) **CELL SORTING APPARATUS**

(30) Priority: 31.05.2023 CN 202310637202; 31.05.2023 CN 202310646102; 31.05.2023 CN 202310637622; 31.05.2023 CN 202310640561; 31.05.2023 CN 202310632744; 31.05.2023 CN 202310637280; 31.05.2023 CN 202310641883; 31.05.2023 CN 202310646155; 31.05.2023 CN 202310632646; 31.05.2023 CN 202310632175
(71) Applicant: Nanjing Livingchip Biotechnology Co., Ltd., Nanjing, Jiangsu 211899 (CN)
(72) Inventor: SUN, Minglei, Nanjing, Jiangsu 211899 (CN)
(74) Representative: Corradini, Cesare
(86) International application number: PCT/CN2023/142804
(87) International publication number: WO 2024/244454

(57) **Abstract**

This disclosure relates to a cell sorting apparatus. The cell sorting apparatus includes: a body; a support mechanism, where the support mechanism includes a first support member configured to carry and place a to-be-sorted member for accommodating to-be-sorted cells; an imaging mechanism, configured to obtain image information of the to-be-sorted cells placed in the support mechanism; a display mechanism, configured to display the image information of the to-be-sorted cells obtained by the imaging mechanism; a picking mechanism, configured to suck a cell meeting a preset requirement in the to-be-sorted member; and a control mechanism, configured to control the suck and release of the cell meeting the preset requirement. The cell meeting the preset requirement are sucked by the picking mechanism, so that screening efficiency of the cell sorting apparatus is improved, and high-throughput cell sorting can be implemented.

## Description

### TECHNICAL FIELD

This disclosure relates to the field of cell sorting, and in particular, to a cell sorting apparatus.

### BACKGROUND

With the development of the biomedical field, cell sorting technologies emerge. A specific cell subpopulation is separated from a mixed cell sample according to characteristics of cells, to implement functional analysis of specific cells, especially single-cell sorting and suction.

In the related art, a single-cell sorting method mainly includes a mouth pipette technology, a limiting dilution technology, a fluorescence-activated cell sorting technology, and the like. However, the mouth pipette technology and the limiting dilution method are low-throughput and high-labor-intensity single-cell separation methods, and the cell sorting precision and accuracy of the fluorescence-activated cell sorting technology are difficult to control, reducing the cell sorting efficiency.

### SUMMARY

According to various embodiments of this disclosure, an aspect of the present disclosure provides a cell sorting apparatus, including: a body; a support mechanism configured to be mounted on the body, the support mechanism includes a first support member configured to carry and place a to-be-sorted member for accommodating to-be-sorted cells, and the first support member configured to move relative to the body, to cause the to-be-sorted member to move to a preset region relative to the body; an imaging mechanism configured to obtain image information of the to-be-sorted cells placed in the support mechanism, where the imaging mechanism is mounted on the body, the imaging mechanism includes an objective lens configured to image the to-be-sorted cells in the preset region; a display mechanism configured to display the image information of the to-be-sorted cells obtained by the imaging mechanism; a picking mechanism configured to suck a cell meeting a preset requirement in the to-be-sorted member, where the picking mechanism is configured to be mounted on the body, the picking mechanism includes a sucking member movable relative to the body, and the sucking member is configured to suck and release the cell meeting the preset requirement; and a control mechanism configured to control movement of the support mechanism relative to the body, control movement of the sucking member relative to the body, and control suck and release of the cell meeting the preset requirement by the sucking member, where the control mechanism is configured to be mounted on the body.

Implementations of this aspect may include one or more of the following features.

In the cell sorting apparatus, the control mechanism first controls the imaging mechanism and the display mechanism to select the cell meeting the preset requirement from the to-be-sorted cells, and then controls the picking mechanism to suck and release the cell meeting the preset requirement, so that the to-be-sorted cells are automatically sucked, released, and recovered, the cell sorting efficiency and accuracy are improved, and high-throughput cell sorting can be implemented.

In one of the embodiments, the support mechanism includes a support assembly and a support movement assembly, the support assembly is configured to carry and place the to-be-sorted member, the support assembly is placed on the support movement assembly, and the support movement assembly is configured to drive the support assembly to move, to place the to-be-sorted member between the sucking member and the objective lens.

In one of the embodiments, the support assembly includes an accommodation unit placed on the support movement assembly, the accommodation unit is configured to recover the cell meeting the preset requirement, and the support movement assembly is configured to drive the accommodation unit to move, to place the accommodation unit between the sucking member and the objective lens.

In one of the embodiments, the cell sorting apparatus further includes a fixing mechanism, the fixing mechanism is configured to fix a biological culture chip to the support mechanism, the biological culture chip is configured as the to-be-sorted member, and the biological culture chip is configured to accommodate the to-be-sorted cells.

In one of the embodiments, the fixing mechanism includes a chip fixing assembly and a support limiting assembly, the chip fixing assembly is configured to be placed on the support assembly, the chip fixing assembly includes a chip accommodation member configured to accommodate the biological culture chip, and the support limiting assembly is arranged on the support assembly and is configured to fix the chip accommodation member onto the support assembly.

In one of the embodiments, the imaging mechanism includes an imaging lighting unit, an optical path assembly, and a detection assembly, the imaging lighting unit is connected to the picking mechanism, the imaging lighting unit is configured to provide lighting for the to-be-sorted member and the optical path assembly, the to-be-sorted member is arranged between the imaging lighting unit and the optical path assembly, the optical path assembly is configured to image the to-be-sorted cells and transfer the image information of the to-be-sorted cells to the detection assembly, the detection assembly is connected to the display mechanism, and the detection assembly is configured to obtain the image information of the to-be-sorted cells obtained by the optical path assembly.

In one of the embodiments, the imaging mechanism further includes a fluorescence assembly, the fluorescence assembly and the optical path assembly are arranged on a same side of the to-be-sorted cells, the fluorescence assembly includes an excitation light source and a filter unit, the filter unit is configured to filter excitation light emitted by the excitation light source, at least one filter unit is configured, and the light emitted by the excitation light source enables the to-be-sorted cells to emit fluorescence through the filter unit.

In one of the embodiments, the picking mechanism further includes a mounting assembly, a picking movement assembly, and a recovery assembly that are mounted on the body, the mounting assembly is configured to mount the sucking member, the picking movement assembly is configured to drive the sucking member to move, to cause the sucking member to be close to or away from the support mechanism, and the recovery assembly is connected to the sucking member, to enable the sucking member to suck the cell meeting the preset requirement.

In one of the embodiments, the picking mechanism further includes a clamping unit, the clamping unit is configured to be mounted on the mounting assembly, the clamping unit is configured to clamp and release the sucking member, the clamping unit includes at least one clamping member movable between a clamping position and a release position, the clamping member clamps at least part of the sucking member in the clamping position, and the clamping member releases the sucking member in the release position.

In one of the embodiments, the clamping unit further includes a first clamping driving member and a clamping body, the clamping body is configured to mount the first clamping driving member and the clamping member, and the first clamping driving member is configured to drive the clamping member to move between the clamping position and the release position.

In one of the embodiments, the clamping member is configured as an elastic member located between the first clamping driving member and the clamping body, and the elastic member is configured to change elastic deformation of the elastic member in a radial direction by adjusting a displacement between the first clamping driving member and the clamping body, to clamp or release the sucking member by the elastic member.

In one of the embodiments, the mounting assembly includes an accommodation member and at least one fixing member supported by the accommodation member, the accommodation member is configured to accommodate the clamping unit, and the fixing member is configured to fix the clamping unit onto the accommodation member.

In one of the embodiments, the picking mechanism further includes a picking lighting unit, the picking lighting unit is connected to the mounting assembly and located at an end of the sucking member away from the first support member, and light emitted by the picking lighting unit is passable through the sucking member.

In one of the embodiments, the sucking member is provided with a hollow pipe, the picking lighting unit includes a selection lighting source, the selection lighting source extends in an axial direction of the sucking member and is configured at an end of the sucking member, and light emitted by the selection lighting source is passable through the hollow pipe of the sucking member to form a sucking member projection.

In one of the embodiments, the cell sorting apparatus further includes a cleaning assembly, where the cleaning assembly includes a cleaning solution unit and a driving unit, the cleaning solution unit is configured to clean the sucking member, a hollow pipe is formed on the sucking member, the cleaning solution unit includes a first cleaning liquid and a second cleaning liquid, the first cleaning liquid is different from the second cleaning liquid, the driving unit is connected to the sucking member, and the driving unit is configured to suck the first cleaning liquid into the hollow pipe to clean the sucking member a first preset quantity of times and suck the second cleaning liquid into the hollow pipe to clean the sucking member a second preset quantity of times. Another aspect of the present disclosure provides a cell sorting apparatus, comprising: a body; a support mechanism configured to be mounted on the body, wherein the support mechanism comprises a first support member configured to hold a to-be-sorted member for accommodating to-be-sorted cells, and the first support member is configured to move relative to the body, to cause the to-be-sorted member to move to a preset region relative to the body; a picking mechanism mounted on the body, configured to suck a cell meeting a preset requirement in the to-be-sorted member, wherein the picking mechanism comprises a sucking member movable relative to the body, a mounting assembly arranged on the body for mounting the sucking member, a picking movement assembly configured to drive the sucking member to move to cause the sucking member to be close to or away from the support mechanism, and a recovery assembly connected to the sucking member, wherein the recovery assembly enables the sucking member to suck the cell meeting the preset requirement; and a control mechanism mounted on the body, configured to control movement of the support mechanism relative to the body, control movement of the sucking member relative to the body, and control suction and release of the cell meeting the preset requirement by the sucking member.

A third aspect of the present disclosure provides a cell sorting apparatus, comprising: a body; a support mechanism configured to be mounted on the body, wherein the support mechanism comprises a first support member configured to hold a to-be-sorted member for accommodating to-be-sorted cells, and the first support member is configured to move relative to the body, to cause the to-be-sorted member to move to a preset region relative to the body; a picking mechanism mounted on the body, configured to suck a cell meeting a preset requirement in the to-be-sorted member, wherein the picking mechanism comprises a sucking member movable relative to the body, a mounting assembly arranged on the body for mounting the sucking member, a picking movement assembly configured to drive the sucking member to move to cause the sucking member to be close to or away from the support mechanism, and a recovery assembly connected to the sucking member, wherein the recovery assembly enables the sucking member to suck the cell meeting the preset requirement; and a control mechanism mounted on the body, configured to control movement of the support mechanism relative to the body, control movement of the sucking member relative to the body, and control suction and release of the cell meeting the preset requirement by the sucking member.

### BRIEF DESCRIPTION OF THE DRAWINGS

To describe the technical solutions in the embodiments of this disclosure or in the conventional technology more clearly, the following briefly describes the accompanying drawings required for describing the embodiments or the conventional technology. Apparently, the accompanying drawings in the following description show merely some embodiments of this disclosure, and a person of ordinary skill in the art may still derive other drawings from these disclosed accompanying drawings without creative efforts.
FIG. 1 is a schematic diagram of a cell sorting apparatus according to some embodiments of this disclosure;
FIG. 2 is an axonometric view of a cell sorting apparatus with a housing assembly removed according to some embodiments of this disclosure;
FIG. 3 is a front view of the cell sorting apparatus in FIG. 2 with a display mechanism removed;
FIG. 4 is an enlarged view of a picking mechanism in FIG. 3;
FIG. 5 is an axonometric view of a picking mechanism in FIG. 4;
FIG. 6 is a right view of a picking mechanism in FIG. 4;
FIG. 7 is a schematic diagram of a sucking member in FIG. 2;
FIG. 8 is an axonometric view of a mounting assembly and a lighting assembly of a cell sorting apparatus according to some embodiments of this disclosure;
FIG. 9 is a full cross-sectional front view of a mounting assembly and a lighting assembly of a cell sorting apparatus according to some embodiments of this disclosure;
FIG. 10 is a full cross-sectional top view of a mounting assembly and a lighting assembly according to some embodiments of this disclosure;
FIG. 11 is an axonometric view of a clamping unit of a sucking member according to some embodiments of this disclosure;
FIG. 12 is a cross-sectional view of a clamping unit of a sucking member according to some embodiments of this disclosure;
FIG. 13 is an axonometric view of a first clamping driving member of the clamping unit in FIG. 11;
FIG. 14 is an axonometric view of a clamping body of the clamping unit in FIG. 11;
FIG. 15 is a cross-sectional view of a clamping body of the clamping unit in FIG. 11;
FIG. 16 is an axonometric view of a second clamping driving member of the clamping unit in FIG. 11;
FIG. 17 is a top view of an accommodation member for accommodating a clamping unit in FIG. 8;
FIG. 18 is a cross-sectional view of an accommodation member for accommodating a clamping unit in FIG. 8;
FIG. 19 is an axonometric view of a support mechanism according to some embodiments of this disclosure;
FIG. 20 is a front view of the support mechanism in FIG. 19;
FIG. 21 is an axonometric view of a support assembly in the support mechanism in FIG. 19;
FIG. 22 is a front view of a first support member in the support mechanism in FIG. 19;
FIG. 23 is an axonometric view of a second support member and a first support movement unit in the support mechanism in FIG. 19;
FIG. 24 is an axonometric view of a third support member and a second support movement unit in the support mechanism in FIG. 19;
FIG. 25 is an axonometric view of an accommodation unit of a cell sorting apparatus according to some embodiments of this disclosure;
FIG. 26 is an axonometric view of a reaction tube of the accommodation unit in FIG. 25;
FIG. 27 is an axonometric view of an imaging mechanism of a cell sorting apparatus according to some embodiments of this disclosure;
FIG. 28 is a schematic diagram of imaging of an imaging mechanism of a cell sorting apparatus according to some embodiments of this disclosure;
FIG. 29 is a schematic diagram of control of a control mechanism of a cell sorting apparatus according to some embodiments of this disclosure;
FIG. 30 is a schematic diagram of a biological culture chip according to some embodiments of this disclosure;
FIG. 31 is a schematic diagram of an accommodation cavity of a biological culture chip according to some embodiments of this disclosure;
FIG. 32 is a schematic diagram of an accommodation cavity of a biological culture chip according to some embodiments of this disclosure, where an opening diameter of the accommodation cavity is less than a bottom diameter of the accommodation cavity;
FIG. 33 is a schematic diagram of a chip fixing assembly of a cell sorting apparatus according to some embodiments of this disclosure;
FIG. 34 is a schematic diagram of a pressure applying member according to some embodiments of this disclosure;
FIG. 35 is a cross-sectional view of a clamping unit according to some embodiments of this disclosure, where a spacer, a second clamping driving member, and a clamping body are integrally formed;
FIG. 36 is a full cross-sectional top view of a mounting assembly according to some embodiments of this disclosure, where a fixing unit includes a fixing member; and
FIG. 37 is a schematic diagram of operations of a sucking member projection mapping and a projection mark of a sucking member projection recognition portion according to some embodiments of this disclosure;

### DETAILED DESCRIPTION OF THE EMBODIMENTS

The technical solutions in embodiments of this disclosure are clearly and completely described in the following with reference to the accompanying drawings in the embodiments of this disclosure. Apparently, the described embodiments are merely some rather than all of the embodiments of this disclosure. All other embodiments obtained by a person of ordinary skill in the art based on the embodiments of this disclosure without creative efforts shall fall within the protection scope of this disclosure.

In the description of this disclosure, it should be understood that if terms such as "center", "longitudinal", "transverse", "length", "width", "thickness", "on", "below", "front", "back", "left", "right", "vertical", "horizontal", "top", "bottom", "inside", "outside", "clockwise", "anticlockwise", "axial direction", "radial direction", and "circumferential direction" occur, orientation or position relationships indicated by the terms are based on orientation or position relationships shown in the accompanying drawings, and are used only for ease and brevity of illustration and description, rather than indicating or implying that the mentioned apparatus or component must have a particular orientation or must be constructed and operated in a particular orientation. Therefore, such terms should not be construed as limiting of this disclosure.

In addition, if terms such as "first" and "second" occur, the terms are used merely for the purpose of description, and shall not be construed as indicating or implying relative importance or implying a quantity of indicated technical features. Therefore, features defining "first" and "second" can explicitly or implicitly include at least one of the features. In description of this disclosure, if a term such as "multiple" occurs, "multiple" means at least two, such as two and three unless it is specifically defined otherwise.

In this disclosure, unless otherwise explicitly specified or defined, if terms such as "mount", "connect", "connection", and "fix" occur, the terms should be understood in a broad sense. For example, the connection may be a fixed connection, a detachable connection, or an integral connection; may be mechanical connections or electrical connections; or the connection may be a direct connection, an indirect connection through an intermediary, or internal communication between two elements or mutual action relationship between two elements, unless otherwise specified explicitly. A person of ordinary skill in the art may understand the specific meanings of the foregoing terms in this disclosure according to specific situations.

In this disclosure, unless otherwise explicitly specified or defined, if a similar description such as the first feature being located "above" or "below" the second feature occurs, its meaning may be the first feature being in a direct contact with the second feature, or the first feature being in an indirect contact with the second feature through an intermediary. Moreover, the first feature "over", "above" and "up" the second feature may be that the first feature is directly above or obliquely above the second feature, or simply indicates that a horizontal height of the first feature is higher than that of the second feature. The first feature "under", "below" and "down" the second feature may be that the first feature is directly below or obliquely below the second feature, or simply indicates that a horizontal height of the first feature is less than that of the second feature.

It should be noted that, if a component is referred to as "being fixed to" or "being arranged to" another component, the component may be directly on another component, or there may be an intermediate component. If a component is considered to be "connected to" another component, the component may be directly connected to another component, or an intervening component may also be present. If existing, the terms "vertical", "horizontal", "upper", "down", "left", "right" and similar expressions used in this disclosure are only for purposes of illustration but not indicate a unique implementation.

Cell sorting is a technology that separates a cell meeting a preset requirement from to-be-sorted cells according to characteristics of the cells. When functional analysis needs to be performed on certain a cell meeting a preset requirement, for example, researches such as cell culture supernatant analysis and detection of cell molecules and cell co-culture and detection of cell functions are all on the premise of obtaining high-purity a cell meeting a preset requirement.

A cell sorting apparatus is configured to automatically select a cell meeting a preset condition from to-be-sorted cells, to separate and purify the cells, which has advantages such as multi-species, high throughput, multiple screening, a short screening cycle, and high screening accuracy. Further, compared with conventional manual selection, cell sorting by using a cell sorting apparatus can reduce interference from human factors, ensure consistency of cells in a cell sorting process, accurately obtain a single cell with a complete form and relatively high activity, improve the screening efficiency, and implement single-cell high-throughput screening.

A cell sorting apparatus in some embodiments of this disclosure is described in detail below.

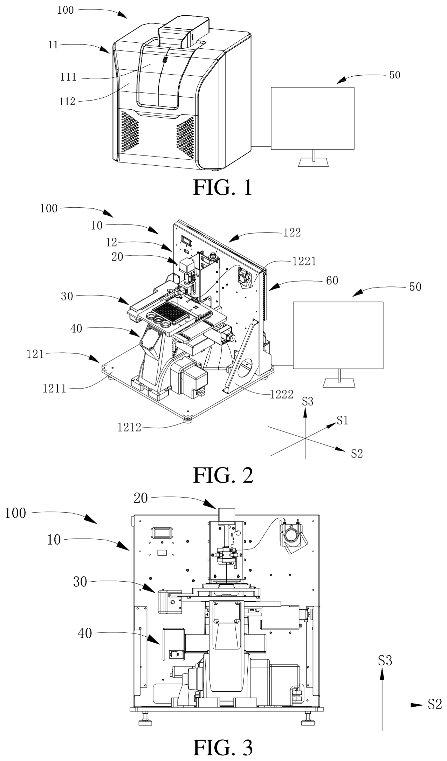

Referring to FIG. 1 to FIG. 3, a schematic diagram of a cell sorting apparatus 100 according to some embodiments of the present disclosure is shown. For ease of comparison and description, a direction perpendicular to a paper surface in FIG. 3 is defined as a first direction S1, a left-right direction is defined as a second direction S2, an up-down direction is defined as a third direction S3, and the first direction S1, the second direction S2, and the third direction S3 are perpendicular to each other.

Referring to FIG. 1 and FIG. 2, a body 10 is a main frame of the cell sorting apparatus 100, and is configured to support and accommodate main components in the cell sorting apparatus 100. The body 10 includes a housing assembly 11 and a support assembly 12. The housing assembly 11 forms an accommodation space of the cell sorting apparatus 100, and the support assembly 12 supports a mechanism arranged in the accommodation space. Referring to FIG. 2 and FIG. 3, in some embodiments, a cell sorting apparatus 100 includes a body 10, a picking mechanism 20, a support mechanism 30, an imaging mechanism 40, a display mechanism 50, and a control mechanism 60. The body 10 is configured to support and accommodate other mechanisms in the cell sorting apparatus 100, to form an accommodation space of the picking mechanism 20, the support mechanism 30, the imaging mechanism 40, and the control mechanism 60. The support mechanism 30 is configured to carry and place a to-be-sorted member, where the to-be-sorted member accommodates to-be-sorted cells. The imaging mechanism 40 is configured to image the to-be-sorted cells and obtain image information of the to-be-sorted cells. The display mechanism 50 is configured to display the image information of the to-be-sorted cells obtained by the imaging mechanism 40, where a cell meeting a preset requirement in the to-be-sorted member are selected after the image information of the to-be-sorted cells is processed. The picking mechanism 20 is configured to suck and recover the cell meeting the preset requirement in the to-be-sorted member, to suck and recover the cell meeting the preset requirement, and complete cell sorting.

The housing assembly 11 is mainly configured to form an accommodation space of an internal mechanism of the cell sorting apparatus 100, to prevent light, foreign objects, and the like from the outside from entering the accommodation space, provide an aseptic environment for a cell sorting process, avoid cell pollution, and ensure cell activity and normal cell culture.

The housing assembly 11 is a hollow structure as a whole, and is configured to accommodate the picking mechanism 20, the support mechanism 30, the imaging mechanism 40, and the control mechanism 60. Generally, the housing assembly 11 is made of a hard material, such as a metal sheet, glass, or hard plastic. A specific material and light transmittance of the housing assembly 11 are set according to a position and a required function of a housing. For example, a housing for overall protection may be made of a metal sheet, and a housing for preventing light from entering may be made of a light-shielding material.

In some embodiments, referring to FIG. 1, the housing assembly 11 includes a first housing 111 and a second housing 112. The first housing 111 is movable relative to the second housing 112 in the second direction S2. Through movement of the first housing 111, the to-be-sorted member can be placed on the support mechanism 30 and the to-be-sorted member can be taken out of the cell sorting apparatus 100.

The first housing 111 is made of a light transmitting material. During use, an operating state of the cell sorting apparatus 100 is observed through the first housing 111. To avoid interference of external light to image information of to-be-sorted cells obtained by the cell sorting apparatus 100 and observe an internal operating state of the cell sorting apparatus 100, the first housing 111 is made of glass, a plastic material, or the like with low light transmittance, such as an acrylic sheet. The second housing 112 is mainly configured to protect internal components of the cell sorting apparatus 100, and is made of an opaque metal sheet or the like as a whole. In some embodiments, the second housing 112 in contact with the first housing 111 is made of a plastic material, to avoid damage to the first housing 111 caused by the second housing 112 when the first housing 111 is opened, and avoid collision damage to the to-be-sorted member and the user during taking. Further, the second housing 112 is further provided with heat dissipation holes for heat dissipation of the cell sorting apparatus 100. The heat dissipation holes dissipate heat generated during operating of the cell sorting apparatus 100, to avoid the impact of the heat on the activity of the to-be-sorted cells, which is conducive to long-term use of the cell sorting apparatus 100, thereby implementing high-throughput screening of the cells.

Referring to FIG. 2 and FIG. 3 again, the support assembly 12 is configured to support main components of the cell sorting apparatus 100, and includes a base 121 and a supporting pedestal 122. The base 121 is configured to carry the main components of the cell sorting apparatus 100. The supporting pedestal 122 is connected to the base 121 in the third direction S3, and is configured to connect and support the main components of the cell sorting apparatus 100.

The base 121 includes a bottom plate 1211 and feet 1212, and the feet 1212 are connected to the bottom plate 1211 in the third direction S3. Relative positions of the feet 1212 relative to the bottom plate 1211 in the third direction S3 are adjusted, to adjust an overall levelness of the cell sorting apparatus 100, so that the cell sorting apparatus 100 can be applied to different desktops, different platforms, and the like. In addition, the feet 1212 further have a vibration resistance function, an anti-skid function, and other functions.

The supporting pedestal 122 includes a support plate 1221 and a rib plate 1222. The support plate 1221 is connected to the bottom plate 1211 in the third direction S3. To obtain as large an accommodation space as possible, the support plate 1221 is arranged on a side of the bottom plate 1211 in the first direction S1. The support plate 1221 is configured to provide connection and support for the main components of the cell sorting apparatus 100 in the third direction S3.

The rib plate 1222 is configured to provide auxiliary support for the support plate 1221, and is in the shape of a right triangle as a whole, where one right side extends in the third direction S3 and is connected to the support plate 1221 in the first direction S 1, and the other right side extends in the first direction S1 and is connected to the bottom plate 1211 in the third direction S3. In some embodiments, the supporting pedestal 122 includes a preset quantity of rib plates 1222. In an embodiment, two rib plates 1222 are arranged opposite to each other on two sides of the bottom plate 1211 in the second direction S2. The arrangement of the rib plates 1222 makes an overall frame of the cell sorting apparatus 100 more stable.

To facilitate assembly and replacement of the support plate 1221, the bottom plate 1211, and the rib plate 1222, the support plate 1221, the bottom plate 1211, and the rib plate 1222 are in a detachable connection to each other. For example, the support plate 1221, the bottom plate 1211, and the rib plate 1222 are connected through fasteners. Generally, the detachable connection includes, but is not limited to, a threaded connection, a screw connection, a snap movable connection, a clamp connection, a flange connection, a pin movable connection, and a socket connection. In another embodiment, the support plate 1221, the bottom plate 1211, and the rib plate 1222 are fixedly connected together. For example, the rib plate 1222 may be fixedly connected to the bottom plate 1211 and the support plate 1221 by welding.

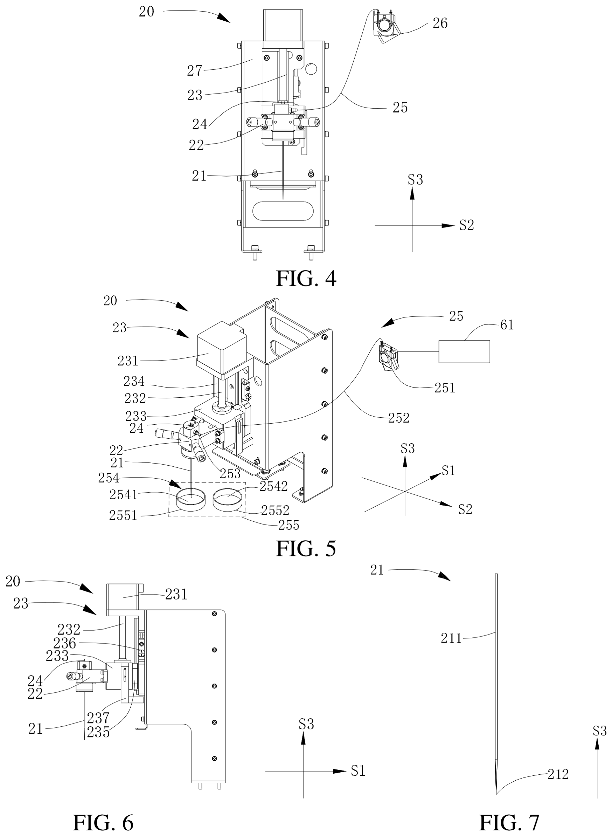

Referring to FIG. 4 to FIG. 6, a picking mechanism according to some embodiments of this disclosure is shown. The picking mechanism 20 includes a sucking member 21, a mounting assembly 22, a picking movement assembly 23, a picking lighting unit 24, a cleaning assembly 25, and a recovery assembly 26. The picking mechanism 20 is configured to select a cell meeting a preset requirement from a to-be-sorted member. Automatic setting of the picking mechanism 20 implements automatic cell suck, thereby improving efficiency of cell sorting, avoiding damage to cells by manual operations, helping implement high-throughput cell sorting, and further implementing single-cell sorting.

With reference to FIG. 7, a sucking member according to some embodiments of this disclosure is shown. The sucking member 21 is configured to suck and release a cell meeting a preset requirement. The sucking member 21 is provided with a hollow pipe 211, to enable cells to enter the sucking member 21 and enable the sucked cells to be stored in the sucking member 21. A size of at least part of the sucking member 21 gradually decreases in a radial direction of the sucking member 21, to form a suck and release opening 212 at an end of the sucking member 21. The size of the suck and release opening 212 matches a size of a cell that needs to be sucked. During suction, cells enter the hollow pipe 211 through the suck and release opening 212, and during release, cells leave the suck and release opening 212 through the hollow pipe 211. The design of the suck and release opening 212 facilitates accurate and effective cell suck, thereby improving the screening efficiency of the cell sorting apparatus 100. A material and a size of the sucking member 21 may be selected according to a size of to-be-sorted cells. For example, a glass capillary tube of 1 millimeter is chosen for the sucking member 21.

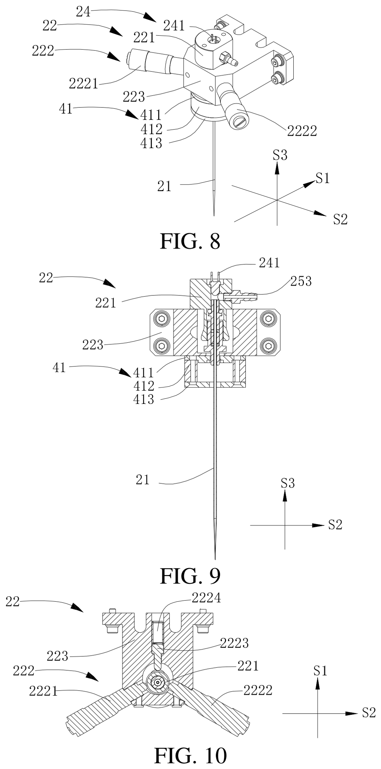

Referring to FIG. 8 to FIG. 10, a mounting assembly and a lighting assembly of a cell sorting apparatus according to some embodiments of this disclosure is shown. The mounting assembly 22 is configured to mount and fix a sucking member 21, to prevent the sucking member 21 from falling off during use. Correspondingly, the mounting assembly 22 may also be configured to replace the sucking member 21. The mounting assembly 22 includes a clamping unit 221, a fixing unit 222, and an accommodation member 223.

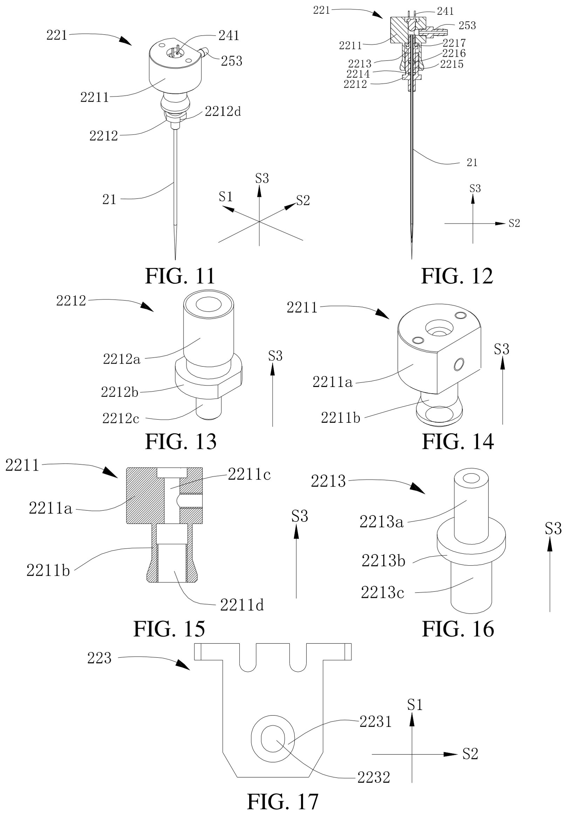

Referring to FIG. 11 and FIG. 12, a clamping unit of a sucking member according to some embodiments of this disclosure is shown. The clamping unit 221 is configured to clamp and release a sucking member 21, and is connected to the sucking member 21 in the third direction S3. With reference to FIG. 7, one end of the sucking member 21 is configured to suck and release to-be-sorted cells, and has a suck and release opening 212 formed. The other end of the sucking member 21 is clamped or released by the clamping unit 221. Through clamping of the clamping unit 221, the sucking member 21 can be stably used, and through release of the clamping unit 221, the sucking member 21 can be replaced. The clamping unit 221 is arranged to fix or release the sucking member 21 without damaging the sucking member 21.

Further, the clamping unit 221 includes at least one clamping member movable between a clamping position and a release position, and the clamping member is configured to clamp the other end of the sucking member. In the clamping position, the clamping member clamps the other end of the sucking member 21, to mount and fix the sucking member 21. In the release position, the clamping member releases the other end of the sucking member 21, to complete recovery of the sucking member 21.

In some embodiments, the clamping unit 221 includes a clamping body 2211 and a first clamping driving member 2212. The first clamping driving member 2212 is configured to drive the clamping member to move between the clamping position and the release position. The clamping body 2211 is configured to support the first clamping driving member 2212. The first clamping driving member 2212 is further provided with a hole for the sucking member 21 to pass through. A hole diameter of at least part of the first clamping driving member 2212 is slightly greater than an outer diameter of the sucking member 21, making it convenient for the sucking member 21 to pass through the first clamping driving member 2212.

In some embodiments, the clamping body 2211 and the first clamping driving member 2212 are in a detachable connection to each other, to facilitate mounting and replacement of the sucking member 21. Specifically, the clamping body 2211 and the first clamping driving member 2212 may be in a threaded connection to each other. The clamping body 2211 is provided with an internal thread, the first clamping driving member 2212 is provided with an external thread, and the connection between the first clamping driving member 2212 and the clamping body 2211 is implemented through the connection between the internal thread and the external thread. The threaded connection is convenient and reliable, and facilitates mounting and detachment of the first clamping driving member 2212.

In another embodiment, there may be an interference fit between the clamping body 2211 and the first clamping driving member 2212. The interference fit improves stability of assembly of the clamping body 2211 and the first clamping driving member 2212, thereby facilitating fixing of the clamping member to the sucking member 21. An outer diameter of at least part of the first clamping driving member 2212 is not less than an inner diameter of the clamping body 2211, to implement an interference fit between the first clamping driving member 2212 and the clamping body 2211.

In some embodiments, at least part of the first clamping driving member 2212 is elastically deformable. When the first clamping driving member 2212 is connected to the clamping body 2211, the first clamping driving member 2212 moves from the release position to the clamping position, an outer wall of the first clamping driving member 2212 comes into contact with and squeezes an inner wall of the clamping body 2211, and the first clamping driving member 2212 is elastically deformed, so that an inner wall of the first clamping driving member 2212 comes into contact with and squeezes an outer wall of the sucking member 21, to clamp and fix the sucking member 21 in the clamping unit 221. In this embodiment, the first clamping driving member 2212 is configured as a clamping member.

In another embodiment, a size of an inner wall of at least part of the first clamping driving member 2212 can be changed. For example, the inner wall of the first clamping driving member 2212 is configured as a cam surface. When the first clamping driving member 2212 is connected to the clamping body 2211, the first clamping driving member 2212 moves from the release position to the clamping position, and the size of the inner wall of the first clamping driving member 2212 is changed, so that an inner wall of the first clamping driving member 2212 comes into contact with and squeezes an outer wall of the sucking member 21, to fix the sucking member 21 in the clamping unit 221. In this embodiment, the first clamping driving member 2212 is configured as a clamping member.

In some embodiments, the clamping unit 221 includes a first clamping driving member 2212, a second clamping driving member 2213, and a clamping body 2211. The second clamping driving member 2213 is configured for the sucking member 21 to thread, and an inner diameter of at least part of the second clamping driving member 2213 is slightly greater than an outer diameter of the sucking member 21, to accommodate the sucking member 21. The second clamping driving member 2213 is arranged between the first clamping driving member 2212 and the clamping body 2211 in the third direction S3. The arrangement of the first clamping driving member 2212 and the second clamping driving member 2213 allows at least two parts of the sucking member 21 to be accommodated, thereby improving the mounting stability of the sucking member 21.

In some embodiments, the second clamping driving member 2213 is fixedly connected to the clamping body 2211. For example, the second clamping driving member 2213 is welded to the clamping body 2211 or the second clamping driving member 2213 and the clamping body 2211 are integrally formed. In another embodiment, a binding manner of the second clamping driving member 2213 and the clamping body 2211 may be one or more of gap binding, interference binding, elastic binding, and surface binding. The second clamping driving member 2213 and the clamping body 2211 may alternatively be in a threaded connection to each other. It may be understood that a connection manner of the second clamping driving member 2213 and the clamping body 2211 is not limited as long as the second clamping driving member 2213 can be accommodated and placed.

In some embodiments, one end of the second clamping driving member 2213 in the third direction S3 is accommodated in the first clamping driving member 2212, an inner diameter of at least part of the first clamping driving member 2212 is slightly greater than an outer diameter of the second clamping driving member 2213, the other end of the second clamping driving member 2213 in the third direction S3 is accommodated in the clamping body 2211, and an inner diameter of at least part of the clamping body 2211 is slightly greater than an outer diameter of the second clamping driving member 2213. At least two parts of the second clamping driving member 2213 are accommodated and fixed, which is conducive to mounting stability of the second clamping driving member 2213.

In some embodiments, at least part of the second clamping driving member 2213 is elastically deformable. When the second clamping driving member 2213 is connected to the first clamping driving member 2212, an outer wall of the second clamping driving member 2213 comes into contact with and squeezes an inner wall of the first clamping driving member 2212, to elastically deform the second clamping driving member 2213, and an inner wall of the second clamping driving member 2213 comes into contact with and squeezes an outer wall of the sucking member 21, to fix the sucking member 21 in the second clamping driving member 2213. In this embodiment, the second clamping driving member is configured as a clamping member.

In some embodiments, a size of an inner wall of at least part of the second clamping driving member 2213 can be changed. For example, the inner wall of the second clamping driving member 2213 is configured as a cam surface. When the second clamping driving member 2213 is connected to the first clamping driving member 2212, a size of an inner wall of the second clamping driving member 2213 is changed, so that the inner wall of the second clamping driving member 2213 comes into contact with and squeezes an outer wall of the sucking member 21, to fix the sucking member 21 in the clamping unit 221. In this embodiment, the second clamping driving member 2213 is configured as a clamping member.

In some embodiments, the clamping unit 221 includes a first clamping driving member 2212, a clamping body 2211, and a first elastic member 2214. The first elastic member 2214 is arranged between the first clamping driving member 2212 and the clamping body 2211 in the third direction S3. An inner diameter of the first elastic member 2214 in a natural state is slightly greater than an outer diameter of the sucking member 21. In this embodiment, the first elastic member 2214 is configured as a clamping member. The elastic deformation of the first elastic member 2214 in the radial direction is changed by adjusting the displacement between the first clamping driving member 2212 and the clamping body 2211, so that the first elastic member 2214 clamps or releases the sucking member 21. Specifically, the first clamping driving member 2212 is provided with an external thread, and the clamping body 2211 is provided with an internal thread. The elastic deformation of the first elastic member 2214 in the radial direction is changed by adjusting tightening or loosening of the external thread and the internal thread, so that the first elastic member 2214 clamps or releases the sucking member 21.

In some embodiments, the clamping unit 221 includes a first clamping driving member 2212, a clamping body 2211, a second clamping driving member 2213, and a first elastic member 2214. The first elastic member 2214 is arranged between the first clamping driving member 2212 and the second clamping driving member 2213 in the third direction S3. When the clamping unit 221 is mounted, elastic deformation of the first elastic member 2214 in a radial direction is changed by adjusting a displacement between the first clamping driving member 2212 and the second clamping driving member 2213. The first elastic member 2214 is configured as a first clamping member. Specifically, opposite end surfaces of the first clamping driving member 2212 and the second clamping driving member 2213 in the third direction S3 jointly squeeze the first elastic member 2214, so that the first elastic member 2214 is deformed in the radial direction, thereby clamping and fixing the sucking member 21.

In this embodiment, the clamping body 2211 or the second clamping driving member 2213 is configured as a first hollow structure for accommodating the sucking member 21, the first clamping driving member 2212 is configured as a second hollow structure for accommodating the sucking member 21, and the first hollow structure, the second hollow structure, and the first clamping member 2214 work together to support and clamp the sucking member 21.

Further, the clamping unit 221 further includes a spacer 2215 arranged between the second clamping driving member 2213 and the first elastic member 2214 in the third direction S3. The spacer 2215 is configured for the sucking member 21 to thread, that is, an inner diameter of the spacer 2215 is slightly greater than an outer diameter of the sucking member 21. The arrangement of the spacer 2215 allows at least three parts of the sucking member 21 to be accommodated, so that the second clamping driving member 2213 is applicable to the mounting of different sucking members 21.

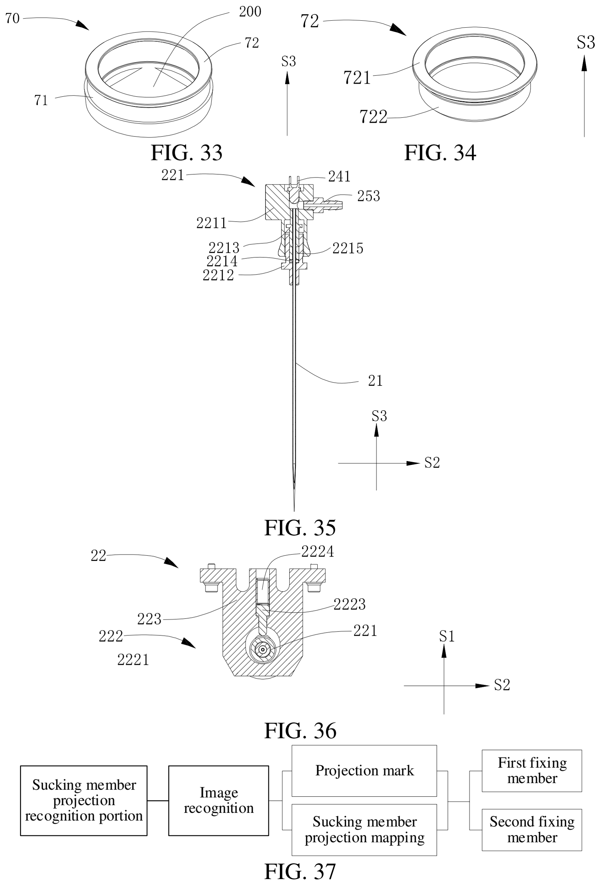

In some embodiments, referring to FIG. 35, a spacer 2215, a second clamping driving member 2213, and a clamping body 2211 are integrally formed. By adjusting a displacement between a first clamping driving member 2212 and the clamping body, a displacement between the spacer 2215 and a first elastic member 2214 is changed, and elastic deformation of the first elastic member 2214 in the radial direction is changed, so that the first clamping member 2214 clamps or releases a sucking member 21.

Further, a clamping unit 221 is provided with a first elastic member 2214 and a second elastic member 2216 spaced apart in the third direction S3. The first elastic member 2214 and the second elastic member 2214 are arranged at two ends of the spacer 2215 in the third direction S3. Specifically, the first elastic member 2214 is configured to be located between the first clamping driving member 2212 and the spacer 2215, and the second elastic member 2216 is configured to be located between the second clamping driving member 2213 and the spacer 2215. The displacement between the first clamping driving member 2212 and the spacer 2215 is adjusted to change the elastic deformation of the first elastic member 2214 in the radial direction, and the displacement between the second clamping driving member 2213 and the spacer 2215 is adjusted to change the elastic deformation of the second elastic member 2216 in the radial direction, so that the first elastic member 2214 and the second elastic member 2216 clamp or release the sucking member 21. In this embodiment, the first elastic member 2214 is configured as a first clamping member, and the second elastic member 2216 is configured as a second clamping member. The arrangement of the first elastic member 2214 and the second elastic member 2216 enables at least two parts of the sucking member 21 to be clamped, thereby ensuring the mounting stability of the sucking member 21.

Specifically, the first clamping driving member 2212 is provided with an external thread, and the clamping body 2211 is provided with an internal thread. The elastic deformation of the first clamping member 2214 and the second clamping member 2216 in the radial direction is changed by adjusting tightening or loosening of the external thread and the internal thread, so that the first clamping member 2214 and the second clamping member 2216 clamp or release the sucking member.

The second clamping driving member 2213 is configured as a first hollow structure for accommodating the sucking member 21, the first clamping driving member 2212 is configured as a second hollow structure for accommodating the sucking member 21, the spacer 2215 is configured as a third hollow structure for accommodating the sucking member 21, and the first hollow structure, the second hollow structure, the third hollow structure, the first clamping member 2214, and the second clamping member 2216 work together to support and clamp the sucking member 21. Specifically, in some embodiments, referring to FIG. 13, a first clamping driving member of a clamping unit according to some embodiments of this disclosure is shown. The first clamping driving member 2212 includes a first clamping driving portion 2212a, an operation portion 2212b, and a second clamping driving portion 2212c. The first clamping driving portion 2212a is provided with a through-hole, to accommodate at least part of a second clamping driving member 2213, a first elastic member 2214, a spacer 2215, and a second elastic member 2216. The first clamping driving portion 2212a is provided with an external thread, and the first clamping driving member 2212 is connected to an internal thread of the clamping body 2211 through the external thread of the first clamping driving portion 2212a. An inner diameter of the first clamping driving portion 2212a is slightly greater than an outer diameter of at least part of the second clamping driving member 2213, and an outer diameter of at least part of the first clamping driving portion 2212a is slightly greater than an inner diameter of at least part of the clamping body 2211. The second clamping driving portion 2212c is configured to accommodate a sucking member 21, and an inner diameter of the second clamping driving portion 2212c is slightly greater than an outer diameter of the sucking member 21.

The operation portion 2212b protrudes from the first clamping driving portion 2212a and the second clamping driving portion 2212c in the radial direction, and is arranged between the first clamping driving portion 2212a and the second clamping driving portion 2212c. An inner diameter of the operation portion 2212b is slightly greater than the outer diameter of the sucking member 21. The operating portion 2212b is configured to collaborate with an external device, and the first clamping driving member 2212 and the clamping body 2211 are connected by the operating portion 2212b. Specifically, an outer peripheral surface of the operation portion 2212b includes opposite arc portions and opposite plane portions, and the plane portions are configured to collaborate with an external device such as a wrench, to connect the external thread of the first clamping driving member 2212 to the internal thread of the clamping body 2211, thereby clamping the sucking member 21.

In some embodiments, referring to FIG. 14 and FIG. 15, a clamping body of a clamping unit according to some embodiments of this disclosure is shown. The clamping body 2211 includes a first clamping body portion 2211a and a second clamping body portion 2211b connected to the first clamping body portion 2211a. The arrangement of the first clamping body portion 2211a is configured to connect a hollow pipe 211 of a sucking member 21 to a recovery assembly 26, to clean the sucking member 21 and suck and release a cell meeting a preset requirement. The first clamping body portion 2211a is provided with a first accommodation cavity 2211c, which is configured to accommodate at least part of the second clamping driving member 2213, to implement communication between the first accommodation cavity 2211c and the hollow pipe 211 of the sucking member 21, and is further configured to connect to at least part of the picking lighting unit 24, to implement lighting of the hollow pipe 211 of the sucking member 21. Specifically, the first accommodation cavity 2211c includes a binding portion, and the binding portion is in a step shape and is configured to connect to the picking lighting unit 24, to implement lighting of the sucking member 21. A central axis of the binding portion coincides with a central axis of the sucking member 21, to ensure a lighting effect of the picking lighting unit 24 on the sucking member 21. It may be understood that, as long as the connection to the picking lighting unit 24 can be implemented, a shape of the binding portion may be adjusted as required, and is not limited to a step shape. Further, the first clamping body portion 2211a is provided with a connection hole in the radial direction, the connection hole is in communication with the first accommodation cavity 2211c, and the connection hole is configured to connect to the recovery assembly 26.

The second clamping body portion 2211b is provided with a second accommodation cavity 2211d in communication with the first accommodation cavity 2211c, to connect to the first clamping driving member 2212 and accommodate the second clamping driving member 2213. The first clamping driving member 2212 is configured to at least partially extend into the second accommodation cavity 2211d, so that at least part of the first clamping driving member 2212 can be accommodated and connected to the clamping body 2211. The first clamping driving member 2212 is configured to at least partially extend and protrude from the second accommodation cavity 2211d to form an operation portion 2212b that facilitates operating the first clamping driving member 2212 to move. A surface of at least part of the second clamping body portion 2211b gradually changes in the third direction S3 to form a tapered surface. This arrangement makes it convenient to fix and adjust the clamping unit 221. An inner diameter of at least part of the second clamping body portion 2211b is slightly greater than an outer diameter of the first clamping driving portion 2212a. Specifically, the second clamping body portion 2211b is provided with an internal thread, the second clamping driving portion 2212a is provided with an external thread, and the internal thread is connected to the external thread.

In some embodiments, referring to FIG. 16, a second clamping driving member of a clamping unit according to some embodiments of this disclosure is shown. The second clamping driving member 2213 is a hollow structure, and is configured for a sucking member 21 to thread, to mount and accommodate the sucking member 21. The second clamping driving member 2213 includes a fourth clamping driving portion 2213a, a second flange portion 2213b, and a fifth clamping driving portion 2213c. Inner diameters of the fourth clamping driving portion 2213a, the second flange portion 2213b, and the fifth clamping driving portion 2213c are slightly greater than an outer diameter of the sucking member 21, to thread and accommodate the sucking member 21. With reference to FIG. 12 and FIG. 15, at least part of the fourth clamping driving portion 2213a is accommodated in the first clamping body portion 2211a in the third direction S3, to bind the second clamping driving member 2213 to the clamping body 2211. An outer diameter of the fourth clamping driving portion 2213a is slightly less than an inner diameter of at least part of the first clamping body portion 2211a. With reference to FIG. 12 and FIG. 13, at least part of the fifth clamping driving portion 2213c is accommodated in the first clamping driving portion 2212a, to bind the second clamping driving member 2213 to the first clamping driving member 2212. An outer diameter of the fifth clamping driving portion 2213c is slightly less than an inner diameter of the first clamping driving portion 2212a.

The second flange portion 2213b protrudes from the fourth clamping driving portion 2213a and the fifth clamping driving portion 2213c in the third direction S3, and is arranged between the fourth clamping driving portion 2213a and the fifth clamping driving portion 2213c. With reference to FIG. 12 and FIG. 15, the clamping unit 221 further includes a sealing member 2217 configured to fix the second clamping driving member 2213. The sealing member 2217 is arranged between the second flange portion 2213b and the first clamping body portion 2211a in the third direction S3. A shape and a size of an inner diameter of the sealing member 2217 match a shape and a size of an outer diameter of the fourth clamping driving portion 2213a. In this embodiment, the sealing member 2217 is accommodated in the second accommodation cavity 2211d, and is supported by the second flange portion 2213b. When the clamping unit 221 is mounted, the sealing member 2217 prevents communication between the first accommodation cavity 2211c and the second accommodation cavity 2211d, so that the to-be-sorted cells sucked or released by the sucking member 21 passes through the first accommodation cavity 2211c and then directly passes through the sucking member 21 without passing through the second accommodation cavity 2211d, to prevent the second accommodation cavity 2211d from affecting the to-be-sorted cells sucked or released by the sucking member 21. In another embodiment, the sealing member 2217 may alternatively be accommodated in the first accommodation cavity 2211c, to prevent communication between the first accommodation cavity 2211c and the second accommodation cavity 2211d. The first elastic member 2214, the second elastic member 2216, and the sealing member 2217 are all annular elastic elements such as O-rings. It should be noted that, none of shapes, sizes, quantities, and types of first elastic members 2214, second elastic members 2216, and sealing members 2217 is limited.

Further, to facilitate mounting and replacement of the sucking member 21 and avoid damage to the sucking member 21 during mounting, central axes of the first clamping driving member 2212, the first elastic member 2214, the spacer 2215, the second elastic member 2216, and the second clamping driving member 2213 in the third direction S3 all coincide with the central axis of the sucking member 21 in the third direction S3.

When the clamping unit 221 is mounted, the first elastic member 2214, the spacer 2215, the second elastic member 2216, and the second clamping driving member 2213 are sequentially placed in the first clamping driving member 2212 in the third direction S3. The second clamping driving member 2213 has one end that is away from the second elastic member 2216 and that is accommodated in the first accommodation cavity 2211c of the first clamping body portion 2211a in the third direction S3, and the other end that abuts against the second elastic member 2216 and that is accommodated in an accommodation cavity of the first clamping driving member 2212 in the third direction S3.

When the sucking member 21 is mounted, the sucking member 21 sequentially threads through respective through-holes formed by the first clamping driving member 2212, the first clamping member 2214, the spacer 2215, the second clamping member 2216, and the second clamping driving member 2213 in the third direction S3 until an end surface of the sucking member 21 is flush with an end surface of the second clamping driving member 2213 accommodated in the clamping body 2211. Then, the first clamping driving member 2212 is tightened, so that the first clamping member 2214 and the second clamping member 2216 are elastically deformed in the radial direction to fix the sucking member 21, and the sealing member 2217 is deformed in the radial direction to fix the second clamping driving member 2213 and block the first accommodation cavity 2211c from the second accommodation cavity 2211d. When the sucking member 21 is recovered or replaced, the first clamping driving member 2212 is loosened, to restore the first clamping member 2214 and the second clamping member 2216 to an original state, and then the sucking member 21 is taken out in a direction opposite to the mounting direction.

In some embodiments, referring to FIG. 11, the first clamping driving member 2212 is provided with a clamping mark 2212d. The clamping mark 2212d is arranged on a surface of the first clamping driving member 2212. The clamping mark 2212d is configured to rotate the first clamping driving member 2212 to a preset position to clamp the sucking member 21. The preset position is the clamping position. Because the sucking member is designed by using a glass capillary tube, the clamping mark 2212d is arranged to effectively avoid damage to the glass capillary tube caused by excessively tightening the first clamping driving member 2212. In another embodiment, the clamping mark 2212d and the clamping body 2211 or a mark located on another component correspond to each other, and are in the clamping position when being aligned with each other. Referring to FIG. 8 to FIG. 10 again, in some embodiments, the fixing unit 222 is arranged in a radial direction of the clamping unit 221, and is configured to fix and adjust the clamping unit 221. The fixing unit 222 includes a first fixing member 2221 and a second fixing member 2222. The first fixing member 2221 and the second fixing member 2222 are spaced apart in a circumferential direction of the clamping unit 221 and extend in the radial direction of the clamping unit 221. The first fixing member 2221 and the second fixing member 2222 are arranged to fix at least two parts of the clamping unit 221.

With reference to FIG. 12 and FIG. 15, the first fixing member 2221 and the second fixing member 2222 are spaced apart in a circumferential direction of the second clamping body portion 2211b of the clamping body 2211. Specifically, when the clamping unit 221 is fixed, an end of the first fixing member 2221 in a radial direction of the second clamping body portion 2211b abuts against a tapered surface of the fifth clamping driving portion 2213c, and an end of the second fixing member 2222 in a radial direction of the fifth clamping driving portion 2213c also abuts against the tapered surface of the fifth clamping driving portion 2213c.

Further, the first fixing member 2221 and the second fixing member 2222 are fine adjustment members, are each arranged as a micrometer structure, and are configured to fix the clamping unit 221 and fine adjust the clamping unit 221, so that the sucking member 21 can be in the preset position. Generally, the micrometer structure includes a screw rod, a fixed sleeve, a differential cylinder, and a knob. During fixing, the first fixing member 2221 and the second fixing member 2222 are tightened to fix the clamping unit 221. During fine adjustment, the knob and the differential cylinder of the first fixing member 2221 and the knob and the differential cylinder of the second fixing member 2222 are rotated until the sucking member 21 is in the preset position.

In some embodiments, the fixing unit 222 further includes a third fixing member 2223. The third fixing member 2223 is arranged in the circumferential direction of the clamping unit 221, and extends in the radial direction of the clamping unit 221. The third fixing member 2223 is arranged to support and fix at least three parts of the clamping unit 221.

In some embodiments, the fixing unit further includes an elastic eccentrically pressing element 2224 configured to eccentrically press the third fixing member 2223. One end of the elastic eccentrically pressing element 2224 is connected to the third fixing member 2223. For example, the third fixing member 2223 and the elastic eccentrically pressing element 2224 may be a combination of a spring base and a spring. The third fixing member 2223 abuts against the clamping unit 221, so that an eccentrically pressing force of the elastic eccentrically pressing element 2224 is overcome to move the first fixing member 2221 or the second fixing member 2222, thereby adjusting the position of the clamping unit 221. During fixing, the third fixing member 2223 and the elastic eccentrically pressing element 2224 can provide elastic support for the clamping unit 221, thereby ensuring the mounting stability of the clamping unit 221. Further, the third fixing member 2223 is arranged in the first direction S 1, and the first fixing member 2221 and the second fixing member 2222 are symmetrically arranged on two sides of the third fixing member 2223 in the first direction S1. It may be understood that, as long as the sucking member 21 can be fixed, the quantity and the type of fixing units 222 may be set according to actual requirements, and are not limited herein.

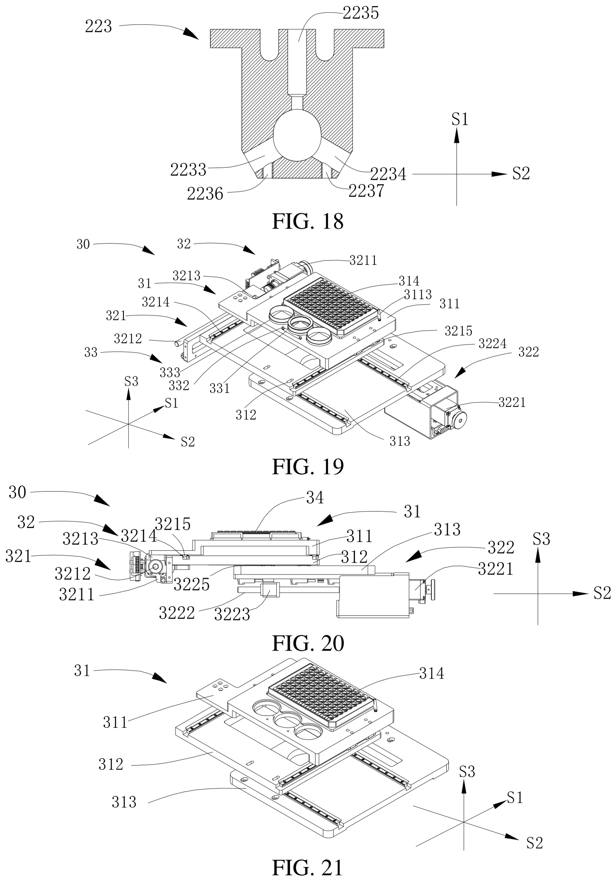

Referring to FIG. 17 and FIG. 18, FIG. 17 and FIG. 18 are schematic diagrams of an accommodation member according to some embodiments of this disclosure. With reference to FIG. 8 to FIG. 10, the accommodation member 223 is a hollow structure, and is configured to accommodate at least part of the clamping unit 221. Two end surfaces of the accommodation member 223 in the third direction S3 are respectively provided with a first hole 2231 and a second hole 2232. The clamping unit 221 sequentially passes through the first hole 2231 and the second hole 2232 in the third direction S3, to place the clamping unit 221. The clamping unit 221 sequentially passes out of the second hole 2232 and the first hole 2231, to take out the clamping unit 221. A size of the first hole 2231 is greater than a size of the clamping unit 221, and a position of the clamping unit 221 relative to the first hole 2231 may be adjusted by moving the first fixing member 2221, or a position of the clamping unit 221 relative to the first hole 2231 may be adjusted by moving the second fixing member 2222.

With reference to FIG. 15, the size of the first hole 2231 is less than an outer diameter of the first clamping body portion 2211a and greater than an outer diameter of the second clamping body portion 2211b, that is, at least part of the clamping body 2211 protrudes from the first hole 2231 in the third direction S3. With reference to FIG. 13, a diameter of the second hole 2232 is less than an outer diameter of the operation portion 2212b and greater than an outer diameter of the second clamping driving portion 2212c, to thread the sucking member 21 and the first clamping driving member 2212. The clamping unit 221 is mounted and accommodated in the accommodation member 223 through the first hole 2231 and the second hole 2232.

The accommodation member 223 is further provided with a first mounting hole 2233, a second mounting hole 2234, and a third mounting hole 2235 for mounting the fixing unit 222. The first mounting hole 2233, the second mounting hole 2234, and the third mounting hole 2235 are all arranged in the radial direction of the clamping unit 221. Specifically, with reference to FIG. 10, the first mounting hole 2233, the second mounting hole 2234, and the third mounting hole 2235 are arranged in a radial direction of a tapered surface of the second clamping body portion 2211b. At least part of the first fixing member 2221 passes through the first mounting hole 2233, enters the first hole 2231, and abuts against the clamping unit 221. At least part of the second fixing member 2222 passes through the second mounting hole 2234, enters the first hole 2231, and abuts against the clamping unit 221. At least part of the third fixing member 2223 passes through the third mounting hole 2235, enters the first hole 2231, and abuts against the clamping unit 221. The elastic eccentrically pressing element 2224 is located in the third mounting hole 2235, and eccentrically presses the third fixing member 2223 toward the center of the first hole 2231.

To fix the first fixing member 2221 and the second fixing member 2222, the accommodation member 223 is further provided with a first fixing hole 2236 and a second fixing hole 2237. Fasteners fix the first fixing member 2221 in the accommodation member 223 through the first fixing hole 2236, and fix the second fixing member 2222 in the accommodation member 223 through the second fixing hole 2237. Referring to FIG. 18, the first fixing hole 2236 and the second fixing hole 2237 are spaced apart on the accommodation member 223 in the second direction S2. Fasteners are screwed into the fixing holes, and abut against the first fixing member 2221 and the second fixing member 2222, to fix the first fixing member 2221 and the second fixing member 2222 on the accommodation member 223. It should be noted that, the structure of the accommodation member 223 may be adjusted according to an actual design, and is not limited to the foregoing structure.

When the clamping unit 221 is mounted, the first fixing member 2221 and the second fixing member 2222 are loosened, the clamping unit 221 is taken out of the accommodation member 223 in the third direction S3, and the sucking member 21 is mounted into the clamping unit 221. The foregoing process of mounting the sucking member 21 into the clamping unit 221 is repeated, so that the sucking member 21 is fixedly mounted on the clamping unit 221. Then, the clamping unit 221 on which the sucking member 21 is mounted is placed in the accommodation member 223 in the third direction S3, the first fixing member 2221 and the second fixing member 2222 are tightened, and an eccentrically pressing force applied to the clamping unit by the third fixing member 2223 and the elastic eccentrically pressing element 2224 needs to be overcome, to mount the clamping unit 221.

It should be noted that, the fixing unit 222 needs to include only one of the above fixing members. In another embodiment, referring to FIG. 36, a fixing unit 222 includes a third fixing member 2223 and an elastic eccentrically pressing element 2224. One end of the elastic eccentrically pressing element 2224 is connected to the third fixing member 2223, and the third fixing member 2223 abuts against a clamping unit 221, so that an eccentrically pressing force of the elastic eccentrically pressing element 2224 is overcome to fix the clamping unit 221 to an accommodation member 223, to mount and fix the clamping unit 221.

Referring to FIG. 4 to FIG. 6 again, in some embodiments, the picking movement assembly 23 is configured to drive the sucking member 21 to move relative to the body 10 in the third direction S3, to suck and release the cells. The picking movement assembly 23 includes a selection driving member 231, a selection transmission member 232, and a selection movement member 233. Under the control of the control mechanism 60, the selection driving member 231 drives the selection transmission member 232 to rotate, to move the selection movement member 233 in the third direction S3. With reference to FIG. 8, the accommodation member 223 is connected to the selection movement member 233 in the first direction S1. Under the driving of the selection driving member 231 and the rotation of the selection transmission member 232, the selection movement member 233 drives the sucking member 21 to move in the third direction S3. Specifically, in an embodiment, the selection driving member 231 is a stepper motor, to quantitatively move the sucking member 21 and ensure movement accuracy of the sucking member 21. The displacement accuracy of the sucking member 21 in this embodiment is 2 micrometers. The selection transmission member 232 is a screw rod, and extends in the third direction S3, to move the sucking member 21 in the third direction S3.

The accommodation member 223 is detachably connected to the selection movement member 233 by a fastener. To facilitate the mounting of the accommodation member 223, the selection movement member 233 is provided with a binding groove. The arrangement of the combination groove makes it convenient to position and mount the accommodation member 223 on the selection movement member 233. It may be understood that, as long as the picking movement assembly 23 can drive the mounting assembly 22 to move, a connection manner of the selection movement member 233 and the mounting assembly 22 is not limited.

In some embodiments, the picking movement assembly 23 further includes a selection guide member 234 and a selection sliding member 235. The selection guide member 234 extends in the third direction S3, and the selection sliding member 235 is arranged on the selection guide member 234 and is connected to the selection movement member 233, to move the selection movement member 233 in the third direction S3. The arrangement of the selection guide member 234 and the selection sliding member 235 makes it convenient to move the selection movement member 233. It should be noted that, extension lengths of the selection guide member 234 and the selection transmission member 232 are determined according to a movement distance of the sucking member, and are not limited herein.

In some embodiments, the picking movement assembly 23 further includes a selection displacement sensor 236 and a selection limiting member 237. The selection displacement sensor 236 is arranged on a side of the selection guide member 234 in the second direction S2. The arrangement of the selection displacement sensor 236 makes it convenient to obtain a movement distance of the sucking member 21, and helps the control mechanism 60 control movement of the sucking member 21. It should be noted that, a type and a mounting position of the selection displacement sensor 236 are not limited as long as a displacement signal can be transmitted.

The selection limiting member 237 extends in the third direction S3 and is connected to the selection movement member 233. The selection limiting member 237 is arranged on a side of the selection guide member 234 in the second direction S2. The selection limiting member 237 and the selection displacement sensor 236 may be arranged on a same side of the selection guide member 234, or may be respectively arranged on two sides of the selection guide member 234. The arrangement of the selection limiting member 237 limits a movement distance of the sucking member 21 in the third direction S3, and a highest position and a lowest position of the sucking member 21 may be obtained through the selection displacement sensor 236, so that the sucking member 21 can be prevented from coming into contact with the support mechanism 30 during movement and being damaged.

Under the control of the control mechanism 60, the selection driving member 231 drives the selection movement member 233 to move along the selection guide member 234 in the third direction S3, to drive the sucking member 21 connected to the selection movement member 233 to move in the third direction S3, thereby enabling the sucking member 21 to approach and move away from the to-be-sorted cells and enabling the sucking member 21 to suck and release the to-be-sorted cells.

Referring to FIG. 8 and FIG. 9 again, the picking lighting unit 24 is connected to the mounting assembly 22 and is located at an end of the sucking member 21 away from the support mechanism 30. The picking lighting unit 24 includes a picking lighting source 241 connected to the control mechanism 60. The picking lighting source 241 and the sucking member 21 are arranged in the third direction S3, and light emitted by the picking lighting source 241 can pass through the hollow pipe 211 of the sucking member 21 to form a projection of the sucking member 21, thereby facilitating positioning of the sucking member 21. With reference to FIG. 15, the picking lighting source 241 is arranged in the first accommodation cavity 2211c of the clamping body 2211.

To ensure that the sucking member 21 can suck and release cells, it is necessary to connect the picking lighting source 241 hermetically and the first accommodation cavity 2211c to each other, to block the first accommodation cavity 2211c from the external environment. In an embodiment, the picking lighting source 241 is fixed in the first accommodation cavity 2211c of the first clamping body portion 2211a by gluing. The gluing connection is convenient to operate, and can ensure sealing performance between the picking lighting source 241 and the clamping body 2211, so that the sucking member 21 can suck cells under the driving of the recovery assembly 26. It may be understood that, as long as the picking lighting source 241 is fixed, a connection manner of the picking lighting source 241 and the clamping body 2211 is not limited.

In some embodiments, the picking lighting source 241 is an LED light source, and is fixedly connected to the clamping body 2211. A central axis of the LED light source is parallel to or aligned with the central axis of the sucking member 21, so that light emitted by the LED light source can pass through the hollow pipe 211 of the sucking member 21. The LED light source has high lighting efficiency and a long service life, and is convenient to mount, thereby ensuring long-term use of the cell sorting apparatus 100. It should be noted that, a position of the picking lighting source 241 is set according to a position of the sucking member 21. As long as light can be emitted, a type of the picking lighting source 241 is not limited to an LED light source.

In a feasible embodiment, a sealing element is further arranged between the picking lighting source 241 and the first clamping body portion 2211a. The sealing element needs to enable light to pass through the hollow pipe 211 of the sucking member 21. The arrangement of the sealing element provides buffering for the picking lighting source 241 and further ensures the sealing performance between the sucking member 21 and the clamping body 2211.

In some embodiments, with reference to FIG. 37, the cell sorting apparatus 100 includes a sucking member projection recognition portion, and the sucking member projection recognition portion is configured to recognize a sucking member projection and form a sucking member projection mapping. A projection mark is arranged in the sucking member projection mapping. The sucking member projection can be adjusted through the first fixing member 2221 and the second fixing member 2223, to change a position of the sucking member projection mapping relative to the projection mark, so that the projection mark is in a preset position of the sucking member projection mapping to complete calibration of the sucking member 21.

During use, the control mechanism 60 controls the picking lighting source 241 to emit light. The light passes through the hollow pipe 211 of the sucking member 21, and passes out of the sucking member 21 through the suck and release opening 212. A sucking member projection is obtained according to the light passing through the suck and release opening 212, and image information (for example, a circular aperture) of the sucking member projection is obtained through the imaging mechanism 40. Based on the image information and a projection mark (for example, a cross cursor) located in the image information the first fixing member 2221 or the second fixing member 2223 is moved to adjust a position of the cross cursor in the circular aperture, for example, moved to a middle position of the cross cursor in the circular aperture. After the position of the sucking member projection is marked, the position is used as a reference, making it convenient for the control mechanism 60 to subsequently control the picking movement assembly 23 to drive the sucking member 21 to move in the third direction S3, to suck a cell meeting the preset requirement.

Referring to FIG. 4 and FIG. 5 again, the cleaning assembly 25 is configured to clean the sucking member 21 before use, so that the activity of the cells can be ensured during the cell suck and release without damaging the normal state of the cells. The cleaning assembly 25 includes a driving unit 251 and a cleaning solution unit 254. The driving unit 251 is connected to the hollow pipe 211 of the sucking member 21 through a communication member 252, so that an air pressure of the hollow pipe 211 of the sucking member 21 can be changed, thereby enabling a cleaning liquid to enter the hollow pipe 211 of the sucking member 21, to clean the sucking member 21. With reference to FIG. 2, the driving unit 251 is arranged on the support plate 1221 in the first direction S1. To facilitate the suck of the cleaning liquid, the driving unit 251 is connected to the sucking member 21 in the third direction S3.

In some embodiments, the driving unit 251 is a pump, and a diaphragm inside the pump is caused to reciprocate through a mechanical apparatus, to compress and stretch air in a cavity of the pump to form a negative pressure, so that a pressure difference is generated between the suck and release opening 212 of the sucking members 21 and an external atmospheric pressure, and the cleaning liquid is sucked into the sucking member 21 under the action of the pressure difference. With reference to FIG. 4, FIG. 12, and FIG. 15, the driving unit 251 is connected to the connection hole of the clamping body 2211, and the clamping body 2211 and the sucking member 21 form a cleaning cavity for cleaning the hollow pipe 211 of the sucking member 21.

In some embodiments, referring to FIG. 4 and FIG. 5, to facilitate the connection between the driving unit 251 and the clamping body 2211, the cleaning assembly 25 further includes a communication member 252. The communication member 252 has one end connected to the driving unit 251 and the other end connected to the connection hole of the clamping body 2211, to form a path with the hollow pipe 211 of the sucking member 21, so that a pressure difference is generated at the suck and release opening 212. Specifically, the communication member 252 may be a plastic hose.

Further, to facilitate the connection between the clamping body 2211 and the communication member 252, the cleaning assembly 25 further includes a passage connection member 253. The passage connection member 253 has one end connected to the clamping body 2211 through the connection hole and the other end enabling the communication member 252 to be sleeved on the passage connection member 253. The passage connection member 253 may be a pagoda connector, has one end in a threaded connection with the clamping body 2211 and the other end sleeved with the communication member 252, and has good sealing performance with the communication member 252.

The passage connection member 253 is a hollow structure, and forms a passage together with the communication member 252 and the sucking member 21. During cleaning, a cleaning liquid flows through an inner wall of the sucking member 21, an inner wall of the clamping body 2211, and an inner wall of the passage connection member 253, to perform cleaning. There is a threaded connection between the passage connection member 253 and the clamping body 2211. It may be understood that, as long as the connection between the passage connection member 253 and the clamping body 2211 can be implemented, a manner of connecting the passage connection member 253 and the clamping body 2211 is not limited. For example, the passage connection member 253 and the clamping body 2211 may be integrally formed.

In some embodiments, to facilitate placement of the cleaning liquid, the cleaning assembly 25 further includes a holding unit 255 configured to hold the cleaning solution unit 254. The holding unit 255 is located on the support mechanism 30 of the cell sorting apparatus 100. The cleaning solution unit 254 includes a first cleaning liquid 2541 and a second cleaning liquid 2542, and the first cleaning liquid 2541 and the second cleaning liquid 2542 are different from each other. The holding unit 255 includes a first holding member 2551 and a second holding member 2552. The first holding member 2551 is configured to hold the first cleaning liquid 2541, and the second holding member 2552 is configured to hold the second cleaning liquid 2542. The driving unit 251 sucks the first cleaning liquid 2541 into the hollow pipe 211 to clean the sucking member 21 for a first preset quantity of times and sucks the second cleaning liquid 2542 into the hollow pipe 211 to clean the sucking member 21 for a second preset quantity of times.

Specifically, in an embodiment, the first cleaning liquid 2541 is ethanol, the second cleaning liquid 2542 is a phosphate buffered saline (PBS), and the first holding member 2551 and the second holding member 2552 are both biological culture dishes. The ethanol is used to sterilize and disinfect the sucking member 21, and the PBS buffer solution is used to provide an internal environment for cells suitable for cell culture, so that the interior of the sucking member 21 does not affect the activity of the cells. It may be understood that types of the cleaning liquids are not limited as long as the sucking member 21 can be cleaned. For example, the second cleaning liquid 2542 may alternatively be a HEPES (4-(2-hydroxyethyl)-1-piperazineethanesulfonic acid) buffer solution.

Further, the first cleaning liquid 2541 is ethanol with a concentration of 75%, and the second cleaning liquid 2542 is a PBS buffer solution with a concentration of 0.01 M. The ethanol with the concentration of 75% can effectively destroy live bacteria and viruses. It should be noted that, the first cleaning liquid 2541 and the second cleaning liquid 2542 with required concentrations may be obtained through preparation, or the first cleaning liquid 2541 and the second cleaning liquid 2542 with required concentrations may be directly used. Correspondingly, the concentrations of the cleaning liquids may be set according to actual requirements, and are not limited to the foregoing concentrations.

Optionally, to ensure an aseptic environment during cell sorting, the first cleaning liquid 2541 and the second cleaning liquid 2542 need to be filtered before use. Specifically, both the ethanol and the PBS buffer solution are filtered through 0.45 micrometer before use. The size requirement for filtering may be set according to experimental requirements, and is not limited to 0.45 micrometer. For example, filtering is performed at 0.22 micrometer. In a feasible embodiment, before the cleaning, the holding member assembly and the cleaning liquid assembly are sterilized, for example, sterilized by using a sterilization kettle. As long as the holding member and the cleaning liquid can be sterilized and disinfected, a specific sterilization manner is not limited.

Before the sucking member 21 sucks cells, the driving unit 251 is connected to the sucking member 21 first, then the first holding member 2551 is taken, a proper amount of first cleaning liquid 2541 is added, and the first holding member 2551 with the first cleaning liquid 2541 added is spaced apart from the sucking member 21 in the third direction S3. Under the control of the control mechanism 60, the picking movement assembly 23 drives the suck and release opening 212 of the sucking member 21 to move into the first cleaning liquid 2541, and the driving unit 251 drives the sucking member 21 to suck the first cleaning liquid 2541 into the hollow pipe 211 of the sucking member 21 to clean the sucking member 21, and drives the sucking member 21 to release the first cleaning liquid 2541 into the first holding member 2551, where the action of aspirating and releasing the first cleaning liquid 2541 may be performed one or more times and a specific quantity of times is determined according to a preset quantity of times to complete the first step of cleaning, to disinfect and sterilize the hollow pipe 211 of the sucking member 21.

Then, the second holding member 2552 is taken, a proper amount of second cleaning liquid 2542 is added, and the second holding member 2552 with the second cleaning liquid 2542 added is spaced apart from the sucking member 21 in the third direction S3. Under the control of the control mechanism 60, the picking movement assembly 23 drives the suck and release opening 212 of the sucking member 21 to move into the second cleaning liquid 2542, and the driving unit 251 drives the sucking member 21 to suck the second cleaning liquid 2542 into the hollow pipe 211 of the sucking member 21 to clean the sucking member 21, and drives the sucking member 21 to release the second cleaning liquid 2542 into the second holding member 2552, where the action of aspirating and releasing the second cleaning liquid 2542 may be performed one or more times and a specific quantity of times is determined according to a preset quantity of times to complete the second step of cleaning. In the second step of cleaning, the first cleaning liquid 2541 is cleaned, and in addition, a relatively stable ion environment and a pH buffering capacity are provided, to facilitate survival of cells in the sucking member 21.

Specific steps after the sucking member 21 sucks the cells are opposite to specific steps before the sucking member sucks the cells. Specifically, the second holding member 2552 is taken first, a proper amount of second cleaning liquid 2542 is added, and the second holding member 2552 with the second cleaning liquid 2542 added is spaced apart from the sucking member 21 in the third direction S3. Under the control of the control mechanism 60, the picking movement assembly 23 drives the suck and release opening 212 of the sucking member 21 to move into the second cleaning liquid 2542, and the driving unit 251 drives the sucking member 21 to suck the second cleaning liquid 2542 into the hollow pipe 211 of the sucking member 21 to clean the sucking member 21, and drives the sucking member 21 to release the second cleaning liquid 2542 into the second holding member 2552, where the action of aspirating and releasing the second cleaning liquid 2542 may be performed one or more times and a specific quantity of times is determined according to a preset quantity of times to complete the first step of cleaning, to clean the residual cells or cell buffer solution in the sucking member 21.

Then, the first holding member 2551 is taken, a proper amount of first cleaning liquid 2541 is added, and the first holding member 2551 with the first cleaning liquid 2541 added is spaced apart from the sucking member 21 in the third direction S3. Under the control of the control mechanism 60, the picking movement assembly 23 drives the suck and release opening 212 of the sucking member 21 to move into the first cleaning liquid 2541, and the driving unit 251 drives the sucking member 21 to suck the first cleaning liquid 2541 into the hollow pipe 211 of the sucking member 21 to clean the sucking member 21, and drives the sucking member 21 to release the first cleaning liquid 2541 into the first holding member 2551, where the action of aspirating and releasing the first cleaning liquid 2541 may be performed one or more times and a specific quantity of times is determined according to a preset quantity of times to complete the first step of cleaning, to clean the second cleaning liquid 2542, and disinfect and sterilize the hollow pipe 211 of the sucking member 21.

In some embodiments, the control mechanism 60 includes a cleaning control unit 61. The cleaning control unit 61 is configured to control the driving unit 251 to suck the first cleaning liquid 2541 into the hollow pipe 211 to clean the sucking member 21 for a first preset quantity of times and control the driving unit 251 to suck the second cleaning liquid 2542 into the hollow pipe 211 to clean the sucking member 21 for a second preset quantity of times. The cleaning control unit 61 may control the driving unit 251 to clean the sucked first cleaning liquid 2541 back and forth for a plurality of times in the hollow pipe 211 in the third direction S3, and then release the first cleaning liquid into the first holding member 2551; and control the driving unit 251 to clean the sucked second cleaning liquid 2542 back and forth for a plurality of times in the hollow pipe 211 in the third direction S3, and then release the second cleaning liquid into the second holding member 2552.

Referring to FIG. 4 and FIG. 5 again, the recovery assembly 26 is configured to suck and release a cell meeting the preset requirement. The recovery assembly 26 includes a driving unit 251, a communication member 252, and a passage connection member 253. For a specific action relationship and position relationship, reference may be made to the descriptions of the cleaning assembly 25. Details are not described herein again. Under the control of the control mechanism 60, the recovery assembly 26 is arranged to suck and release the cells, which facilitates the cell sorting process and improves the cell sorting efficiency.

During use, the recovery assembly 26 moves a cell meeting the preset requirement to be spaced apart from the sucking member 21 in the third direction S3. Under the control of the control mechanism 60, the picking movement assembly 23 drives the suck and release opening 212 of the sucking members 21 to move to a preset position. In this case, there is a preset distance between the suck and release opening 212 and the cell meeting the preset requirement. The control mechanism 60 controls the driving unit 251 to generate a pressure difference at the suck and release opening 212 of the sucking members 21, thereby aspirating the cell meeting the preset requirement into the hollow pipe 211, and completing the suck of the cell meeting the preset requirement. The preset distance is a distance that enables the sucking member 21 to suck the cell meeting the preset requirement into the hollow pipe 211.

To facilitate the mounting of the picking movement assembly 23, referring to FIG. 4, the picking mechanism 20 further includes a mounting bracket 27. The arrangement of the mounting bracket 27 implements fixed mounting of the picking movement assembly 23. The picking mechanism 20 is mounted on the body 10 through the mounting bracket 27. In some embodiments, the mounting bracket 27 is connected to the imaging mechanism 40 in the third direction S3, and the imaging mechanism 40 is mounted on the body 10. It should be noted that, the mounting bracket 27 may be arranged according to actual needs. For example, the mounting bracket 27 is connected to the support plate 1221 in the first direction S1, to mount the picking mechanism 20 on the body 10. In some embodiments, a first mounting base 271 and a second mounting base 272 are arranged on the mounting bracket 27. The first mounting base 271 is arranged on the mounting bracket 27 in the third direction S3, and is configured to fixedly mount the selection guide member 234. The second mounting base 272 is arranged on the mounting bracket 27 in the first direction S1, is perpendicular to the first mounting base 271, and is configured to place and fix the selection driving member 231.

During use, the picking mechanism 20 is connected to the mounting assembly 22 and the picking movement assembly 23, and the sucking member 21 is mounted and fixed on the mounting assembly 22. The picking movement assembly 23 drives the sucking member 21 to move. Cleaning of the sucking member 21 is completed through the cleaning assembly 25. Then, the picking movement assembly 23 drives the sucking member 21 to move. A cell meeting a preset requirement are sucked and recovered by the sucking member 21 in a preset position through the recovery assembly 26. Finally, after the suck is completed, the picking movement assembly 23 drives the sucking member 21 to move, and cleaning of the sucking member 21 is completed through the cleaning assembly 25.

In some embodiments, referring to FIG. 19 and FIG. 20, a support mechanism according to some embodiments of this disclosure is shown. The support mechanism 30 is configured to carry and place a to-be-sorted member, where the to-be-sorted member accommodates to-be-sorted cells. With reference to FIG. 2, the support mechanism 30 is spaced apart from the picking mechanism 20 in the third direction S3, and is arranged between the imaging mechanism 40 and the picking mechanism 20.

The support mechanism 30 is configured to be mounted on the body 10. In some embodiments, the support mechanism 30 is connected to the supporting pedestal 122 and is directly mounted on the body 10. In some embodiments, the support mechanism 30 is connected to the imaging mechanism, and the imaging mechanism is mounted on the body 10, so that the support mechanism 30 is indirectly mounted on the body 10.

The support mechanism 30 can move the to-be-sorted member to a preset position, so that a cell meeting a preset requirement can be sucked and recovered under the action of the picking mechanism 20. The support mechanism 30 includes a support assembly 31 and a support movement assembly 32. The support assembly 31 is configured to carry the to-be-sorted member, the support assembly 31 is placed on the support movement assembly 32, and the support movement assembly 32 can drive the support assembly 31 to move, so that the cell meeting the preset requirement in the to-be-sorted member are spaced apart from the sucking member 21 in the third direction S3. The arrangement of the support mechanism 30 implements the placement and movement of the to-be-sorted member, so that the cell meeting the preset requirement can be sucked and recovered, thereby improving the efficiency of cell sorting.

In some embodiments, referring to FIG. 21, a support assembly of a support mechanism 30 according to some embodiments of this disclosure is shown. The support assembly 31 includes a first support member 311 and a second support member 312. The first support member 311 and the second support member 312 are spaced apart in the third direction S3, the first support member 311 is movably connected to the second support member 312, the first support member 311 moves in the first direction S1 relative to the second support member 312, and the second support member 312 drives the first support member 311 to move together in the second direction S2.

The first support member 311 is configured to place the to-be-sorted member accommodating the to-be-sorted cells, and the first support member 311 is movable in the first direction S1 relative to the base 121 of the body 10 under the driving of the support movement assembly 32. The second support member 312 is configured to place the first support member 311, and the second support member 312 is movable in the second direction S2 relative to the base 121 of the body 10 under the driving of the support movement assembly 32, so that the to-be-sorted member is moved to a preset region relative to the base 121 of the body 10, to suck and recover the cell meeting the preset requirement in the to-be-sorted member in the preset region. It may be understood that movement directions of the first support member 311 and the second support member 312 may be set according to an actual requirement, and are not limited herein. For example, the first support member 311 can move in the second direction S2 under the driving of the support movement assembly 32, and the second support member 312 can move in the first direction S1 under the driving of the support movement assembly 32.

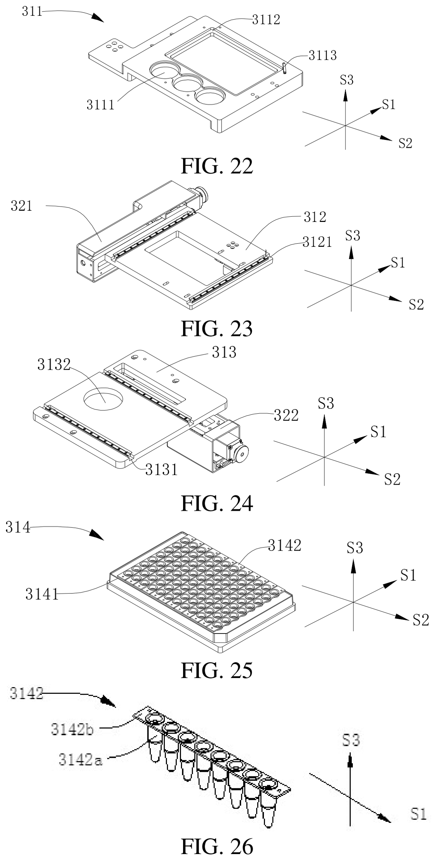

Referring to FIG. 2 and FIG. 22, a first support member according to some embodiments of this disclosure is shown. The first support member 311 is provided with a support portion 3111. The to-be-sorted member can be placed through the support portion 3111. In addition, the imaging mechanism 40 can image, through the support portion 3111, the to-be-sorted cells placed in the to-be-sorted member to obtain image information of the to-be-sorted cells. To make full use of a space of the first support member 311 and improve screening efficiency of the to-be-sorted cells, the first support member 311 is provided with a preset quantity of support portions 3111, to place a preset quantity of to-be-sorted members. For example, the first support member 311 is provided with three support portions 3111 at intervals in the second direction S2, to sort to-be-sorted cells placed in three to-be-sorted members.

In some embodiments, referring to FIG. 21, the support assembly 31 further includes a third support member 313. The third support member 313 is configured to carry and place the first support member 311 and the second support member 312, and is spaced apart from the second support member 312 in the third direction S3. The second support member 312 moves in the second direction S2 relative to the third support member 313.

In some embodiments, referring to FIG. 21, the support assembly 31 further includes an accommodation unit 314 arranged on the first support member 311. The accommodation unit 314 is configured to accommodate and hold a cell meeting a preset requirement, to facilitate subsequent culture of the cell meeting the preset requirement. With reference to FIG. 2, the cell meeting the preset requirement are sucked by the picking mechanism 20 and then may be placed in the accommodation unit 314, and then the accommodation unit 314 is transferred to an incubator, to perform a next step of culture on the cell meeting the preset requirement.

The accommodation unit 314 is a porous structure as a whole. The arrangement of the porous structure can recover and place a plurality of cells, improve the efficiency of cell sorting, and improve the success rate and accuracy of cell sorting. For example, the accommodation unit 314 is provided with 96 holes. It should be noted that, the quantity of holes in the accommodation unit 314 may be set according to actual requirements, and is not limited herein.

Referring to FIG. 25, FIG. 25 shows an accommodation unit according to some embodiments of this disclosure. The accommodation unit 314 includes a support frame 3141 and reaction tubes 3142. The support frame 3141 may be reused, and the reaction tubes 3142 are arranged on the support frame 3141 at intervals in the second direction S2, so that recovery and culture can be performed separately. Optionally, distances between the reaction tubes 3142 are the same. For example, the support frame 3141 is provided with eight reaction tubes 3142 equidistantly in the first direction S1, and is provided with 12 reaction tubes 3142 equidistantly in the second direction S2. It should be noted that, as long as cell recovery and culture can be implemented, a material of the accommodation unit 314 is not limited, and different materials may be selected according to actual requirements. For example, materials of the support frame 3141 and components of accommodation portions 3142a may be the same or different.

The support frame 3141 is provided with through-holes, and the reaction tubes 3142 pass through the through-holes and are bound to the support frame 3141. To facilitate placement of the support frame 3141 and determination of a component position of the accommodation portion 3142a, one of corners of the support frame 3141 is set as a positioning chamfer. Corners of the support frame 3141 other than the positioning chamfer are all set as rounded corners. The arrangement of the rounded corners can prevent the corners of the support frame 3141 from sharpening, which facilitates recovery and culture of the accommodation unit 314. The support frame 3141 is circumferentially provided with a skirt structure extending in the third direction S3. The arrangement of the skirt structure can strengthen the structural strength of the support frame 3141, to prevent the support frame 3141 from being twisted and deformed. Further, the skirt structure is step-shaped to facilitate placement of the support frame 3141.

Referring to FIG. 26, FIG. 26 is a schematic diagram of a reaction tube 3142 according to some embodiments of this disclosure. The reaction tube 3142 includes accommodation portions 3142a and a placement platform 3142b for placing the accommodation portions 3142a. The placement platform 3142b is provided with placement holes corresponding to the accommodation portions 3142a. The arrangement of the accommodation portion 3142a provides an accommodation and culture place for a cell meeting a preset requirement. With reference to FIG. 24, the placement holes on the placement platform 3142b and the through-holes of the support frame 3141 are both in a one-to-one correspondence with the accommodation portions 3142a. The quantity of accommodation portions 3142a matches the quantity of through-holes.

The receiving portion 3142a has one end extending in the third direction S3, where a size of at least part of the tube body gradually increases in the third direction S3 to form a conical shape; and has the other end provided as an opening, to place the cell meeting the preset requirement. The design of the conical shape facilitates cell recovery. In some embodiments, the accommodation portion 3142a is a transparent accommodation portion 3142a, to help subsequently observe the cell meeting the preset requirement.

The placement platform 3142b is in the shape of a rectangular sheet, and two ends of the placement platform 3142b in the first direction S1 are provided with positioning holes that are not on a same axis. This arrangement makes it convenient to determine positions of different accommodation portions 3142a. In some embodiments, the placement platform 3142b and the accommodation portions 3142a are separately injection molded, and then connected by thermosetting to form the reaction tube 3142.

Specifically, in an embodiment, each reaction tube 3142 includes a preset quantity of accommodation portions 3142a arranged in the first direction S1. The quantity of to-be-mounted reaction tubes 3142 may be selected according to specific use requirements, which helps improve use flexibility of the accommodation unit 314, and further helps reduce consumables and avoid waste. For example, the support frame 3141 has 96 mounting through-holes. If 32 accommodation portions 3142a need to be used in a specific experiment, only four reaction tubes 3142 may be mounted into the support frame 3141. It should be noted that, the preset quantity of receiving portions 3142a in each reaction tube 3142 may alternatively be arranged in another manner. For example, a plurality of receiving portions 3142a are distributed in an array or a plurality of receiving portions 3142a are distributed rectangularly.

Referring to FIG. 19 to FIG. 22, the first support member 311 is provided with an accommodation groove 3112 for accommodating the accommodation unit 314. The accommodation unit 314 is placed on the first support member 311 through the placement groove 3112, to place and fix the accommodation unit 314. Specifically, the step-shaped skirt structure of the support frame 3141 collaborates with the placement groove 3112, so that the accommodation unit 314 is placed on the first support member 311. A shape and a size of the placement groove 3112 match a shape and a size of the support frame 3141. Because the accommodation unit 314 is spaced apart from the to-be-sorted member in the first direction S1, the placement groove 3112 provided on the first support member 311 is correspondingly spaced apart from the support portion 3111 in the first direction S1. It may be understood that the positions of the to-be-sorted member and the accommodation unit 314 are not limited as long as the suck and recovery of the cell meeting the preset requirement can be implemented. For example, the to-be-sorted member is spaced apart from the accommodation unit 314 in the second direction S2 or the accommodation unit 314 is spaced apart from the to-be-sorted member in the circumferential direction of the to-be-sorted member. Further, to better fix the first support member 311, the first support member 311 is further provided with an elastic buffer member 3113, the elastic buffer member 3113 is arranged in at least one corner of the placement groove 3112, and the accommodation unit 314 is fixed on the first support member 311 through the elastic buffer member 3113. With reference to FIG. 25 and FIG. 26, to prevent the accommodation portion 3142a from touching the first support member 311 to affect recovery of a cell meeting a preset requirement, the first support member 311 is provided with a through-hole in communication with the placement groove 3112, a size of the through-hole is less than a size of the placement groove 3112, and a step is formed between the through-hole and the placement groove 3112, to place the accommodation unit 314. The size of the through-hole is not limited as long as normal placement of the accommodation portion 3142a can be ensured. Referring to FIG. 19 and FIG. 20 again, the support movement assembly 32 can drive the support assembly 31 to move, to drive the to-be-sorted member to move, to enable the cell meeting the preset requirement in the to-be-sorted member to be sucked, and then drive the accommodation unit 314 to move, to enable the sucked a cell meeting the preset requirement to be released into the accommodation unit 314. With reference to FIG. 2, the control mechanism 60 can control the support movement assembly 32 to movement, and control the picking mechanism 20 to suck and release, achieving suck and release the cell meeting the preset requirement.

In some embodiments, the support movement assembly 32 includes a first support movement unit 321 and a second support movement unit 322. The first support movement unit 321 is configured to drive the support assembly 31 to move in the first direction S1, and the second support movement unit 322 is configured to drive the support assembly 31 to move in the second direction S2. Specifically, the first support movement unit 321 is configured to drive the first support member 311 to move in the first direction S1, and the second support movement unit 322 is configured to drive the second support member 312 to move in the second direction S2.

In some embodiments, the first support movement unit 321 includes a first support driving member 3211, a first support transmission member 3212, and a first support movement member 3213. The first support driving member 3211 can drive the first support transmission member 3212 to rotate to move the first support movement member 3213 in the first direction S1. The first support movement member 3213 is connected to the first support member 311, the first support transmission member 3212 extends in the first direction S1, and under the driving of the first support driving member 3211, the first support movement member 3213 drives the first support member 311 to move in the first direction S1.

Specifically, the first support driving member 3211 may be a stepper motor to quantitatively move the first support member 311, thereby controlling the first support member 311, and the to-be-sorted member and the accommodation unit 314 that are arranged on the first support member 311 to move by a preset distance in the first direction S 1. It may be understood that the type of the first support driving member 3211 is not limited as long as the support assembly 31 can be moved in the first direction S1.

Optionally, the first support driving member 3211 is a five-phase stepper motor. The five-phase stepper motor can implement high-precision positioning of the first support member 311, and can implement high precision that is substantially unchanged during full-step driving through a static angle error during control of a driver for micro-step driving. Compared with other stepper motors, the five-phase stepper motor further implements low-vibration and low-noise driving. The five-phase stepper motor helps high-precision automatic movement of the support assembly 31.

In some embodiments, to facilitate movement of the first support member 311 in the first direction S1, the first support movement unit 321 further includes first support guide members 3214. The first support guide members 3214 extend in the first direction S1, so that the first support member 311 moves in the first direction S1. The first support guide members 3214 are spaced apart on two sides of the second support member 312 in the second direction S2, and the first support guide members 3214 spaced apart can ensure stability during movement of the first support member 311 in the first direction S1. It should be noted that, extension lengths of the first support transmission member 3212 and the first support guide member 3214 are determined by a set movement distance of the first support member 311.

To facilitate the mounting of the first support guide members 3214, referring to FIG. 23, a second support member and a first support movement unit are shown according to some embodiments of this disclosure. The second support member 312 is provided with first support guide grooves 3121, and the first support guide members 3214 are arranged in the first support guide grooves 3121, to ensure stability during movement of the first support member 311. It may be understood that the first support guide members 3214 may alternatively be arranged on a surface of the second support member 312.

In some embodiments, the first support movement unit 321 further includes first carrying sliding members 3215. The first support sliding members 3215 are arranged on the first support guide members 3214 and are connected to the first support member 311. The first support sliding members 3215 slide on the first support guide members 3214 to drive the first support member 311 to move in the first direction S1. The arrangement of the first support sliding members 3215 reduces loss during movement of the first support member 311, to help improve movement stability and displacement accuracy of the first support member 311. Further, each first support guide member 3214 is provided with a preset quantity of first support sliding members 3215, and the first support sliding members 3215 are spaced apart on the first support guide member 3214 in the first direction S1. The preset quantity of first support sliding members 3215 further ensure stability during movement of the first support member 311.

In some embodiments, the first support movement unit 321 further includes a displacement sensor that is not shown in the figure. The displacement sensor may be arranged to obtain position information of the to-be-sorted member and the accommodation unit 314, so that the control mechanism 60 controls the support assembly 31 to move automatically, thereby improving the cell sorting efficiency. Specifically, the displacement sensor is a grating displacement sensor. The grating displacement sensor has high measurement accuracy and resolution, good stability, and a strong anti-interference capability, is convenient to interface with a microcomputer, and is suitable for long-distance transmission. It may be understood that a type of the displacement sensor is not limited as long as position information of the support assembly 31 can be obtained.

In some embodiments, referring to FIG. 19 and FIG. 20, the second support movement unit 322 includes a second support driving member 3221, a second support transmission member 3222, and a second support movement member 3223. The second support driving member 3221 can drive the second support transmission member 3222 to rotate to move the second support movement member 3223 in the second direction S2. The second support movement member 3223 is connected to the second support member 312, the second support transmission member 3222 extends in the second direction S2, and under the driving of the second support driving member 3221, the second support member 312 moves in the second direction S2.

Specifically, the second support driving member 3221 is a stepper motor to quantitatively move the second support member 312, thereby controlling the to-be-sorted member and the accommodation unit 314 to move by a preset distance in the second direction S2. It may be understood that the type of the second support driving member 3221 is not limited as long as the support assembly 31 can be moved in the second direction S2. Optionally, the second support driving member 3221 is also a five-phase stepper motor. The arrangement of the five-phase stepper motor better helps the high-precision automatic movement of the support assembly 31. The movement precision of the support assembly 31 needs to meet the movement requirement of the to-be-sorted member, that is, the suck of adjacent a cell meeting the preset requirement is not interfered with. For example, the movement precision of the support assembly 31 is 2 micrometers.

In some embodiments, the second support movement unit 322 further includes second support guide members 3224. The second support guide members 3224 extend in the second direction S2 and are spaced apart on the third support member 313 in the first direction S1. The arrangement of the second support guide members 3224 makes it convenient to move the second support member 312. It should be noted that, extension lengths of the second support transmission member 3222 and the second support guide member 3224 are determined by a set movement distance of the second support member 312.

Referring to FIG. 24, a third support member and a second support movement unit are shown according to some embodiments of this disclosure. The third support member 313 is provided with second support guide grooves 3131, and the second support guide members 3224 are arranged in the second support guide grooves 3131, to ensure stability during movement of the second support member 312. It may be understood that the second support guide members 3224 and the second support guide grooves 3131 may alternatively be arranged in opposite directions, that is, the second support guide members 3224 are arranged on the third support member 313 and the second support guide grooves 3131 are placed on the second support member.

In some embodiments, the second support movement unit 322 further includes second carrying sliding members 3225. The second support sliding members 3225 are arranged on the second support guide members 3224 and are connected to the second support member 312. The second support sliding members 3225 slide on the second support guide members 3224 to drive the second support member 312 to move, thereby reducing wear during movement of the second support member 312, and improving stability and position accuracy during movement of the second support member 312. Further, each second support guide member 3224 is provided with a preset quantity of second support sliding members 3225, and the second support sliding members 3225 are spaced apart on the second support guide member 3224 in the first direction S1. The preset quantity of second support sliding members 3225 further ensure stability during movement of the second support member 312.

In some embodiments, the second support movement unit 322 further includes a displacement sensor. The displacement sensor is a proximity displacement sensor. The proximity displacement sensor has a simple structure, is stable and reliable, and has high measurement accuracy and high sensitivity. It may be understood that a type and a mounting position of the displacement sensor are not limited as long as the displacement information of the second support member 312 can be obtained.

Referring to FIG. 23 and FIG. 24, the first support movement unit 321 is arranged on a side of the second support member 312 in the second direction S2, and the second support movement unit 322 is arranged on a side of the third support member 313 in the first direction S1. It should be noted that, as long as the movement of the first support member 311 in the first direction S1 and the movement of the second support member 312 in the second direction S2 can be realized, mounting positions of the first support movement unit 321 and the second support movement unit 322 are not limited.

Referring to FIG. 19 again, the support mechanism 30 further includes a support limiting assembly 33. The support limiting assembly 33 is arranged on the support assembly 31, and is configured to fix the to-be-sorted member on the support assembly 31. The support limiting assembly 33 includes an elastic member 331 and eccentrically pressing connection members 332. The elastic member 331 is configured to surround a surface of the to-be-sorted member and eccentrically press the to-be-sorted member in the radial direction of the to-be-sorted member. With reference to FIG. 20, the eccentrically pressing connection members 332 are spaced apart on the first support member 311 in the second direction S2, and the elastic member 331 is sleeved on the eccentrically pressing connection members 332. Specifically, in an embodiment, the eccentrically pressing connection members 332 are arranged in a circumferential direction of the support portion 3111 of the first support member 311, so that the elastic member 331 can eccentrically press and limit the to-be-sorted member placed on the first support member 311. After the to-be-sorted member is placed on the first support member 311, the elastic member 331 is pulled from one side of the support portion 3111 to the other side of the support portion 3111 to eccentrically press the to-be-sorted member, so that the to-be-sorted member abuts against the support portion 3111, thereby ensuring stability during placement and subsequent movement of the to-be-sorted member.

In some embodiments, the support limiting assembly 33 further includes a limiting member 333. The arrangement of the limiting member 333 enables to-be-sorted members of different sizes to be fixed in the support portion 3111. A shape and a size of an inner diameter of the limiting member 333 are substantially the same as a shape and a size of an outer diameter of the to-be-sorted member, so that an outer wall of the to-be-sorted member fits an inner wall of the limiting member 333. A shape and a size of an outer diameter of the limiting member 333 are substantially the same as a shape and a size of a limiting groove in the support portion 3111, so that an outer wall of the limiting member 333 fits the limiting groove in the support portion 3111, to limit the to-be-sorted member on the first support member 311.

Further, in an embodiment, with reference to FIG. 22, the limiting member 333 may be made of a silicone material. The arrangement of the limiting member 333 increases friction between the to-be-sorted member and the support portion 3111, so that the to-be-sorted member is more stable. The elastic member 331 may eccentrically press the limiting member to provide buffering for the to-be-sorted member, thereby avoiding or alleviating damage caused by the elastic member 331 to the to-be-sorted member, and ensuring normal use of the to-be-sorted member. It may be understood that a material, a shape, and a size of the limiting member 333 may be set according to actual needs, and are not limited herein.

In another embodiment, the limiting member 333 may alternatively be a compression member, and a preset quantity of compression members are spaced apart on the limiting groove of the support portion 3111. Through the arrangement of the compression member, the to-be-sorted member is limited in the support portion 3111. The compression member may be a spring, a spring pin, or the like. When the to-be-sorted member is placed in the support portion 3111, the to-be-sorted member presses the compression member in the support portion 3111, and the compression member applies a pressure to the to-be-sorted member while being compressed, to fix the to-be-sorted member in the support portion 3111. It may be understood that the quantity and positions of compression members may be set according to actual needs, and are not limited herein.

In some feasible embodiments, the limiting member 333 may be a fastener. The fastener is arranged on an inner wall of the support portion 3111 to fix the to-be-sorted member in the support portion 3111. The limiting member may alternatively be a clamping member. The clamping member is arranged on a peripheral side of the support portion 3111, so that the to-be-sorted member is fixed in the support portion 3111. It may be understood that a binding manner of the limiting member and the to-be-sorted member is not limited as long as the to-be-sorted member can be fixed in the support portion 3111.

When the support mechanism 30 works, the to-be-sorted member is placed on the support assembly 31, the to-be-sorted member is limited by the support limiting assembly 33, the support movement assembly 32 drives the to-be-sorted member to move to suck a cell meeting a preset requirement, and then drives the accommodation unit 314 to move to recover and culture the cell meeting the requirement.

Specifically, during the suck of the cell meeting the preset requirement, under the control of the control mechanism 60, the first support movement unit 321 drives the to-be-sorted member to move in the first direction S1, and the second support movement unit 322 drives the to-be-sorted member to move in the second direction S2, until the cell meeting the preset requirement are spaced apart from the sucking member 21 in the third direction S3. The picking movement assembly 23 drives the sucking member 21 to move to a preset position, and the driving unit 251 drives the sucking member 21 to suck the cell meeting the preset requirement into the hollow pipe 211, to complete the suck of the cell meeting the preset requirement.

Then, the picking movement assembly 23 drives the suck and release opening 212 of the sucking member 21 to move upward, the first support movement unit 321 drives the accommodation unit 314 to move in the first direction S1, and the second support movement unit 322 drives the accommodation unit 314 to move in the second direction S2, until the corresponding receiving portion 3142a is spaced apart from the sucking member 21 in the third direction S3. The picking movement assembly 23 drives the sucking member 21 to move to a preset position, and the driving unit 251 drives the sucking member 21 to release the cell meeting the preset requirement into the accommodation unit 314, to complete the recovery and culture of the cell meeting the preset requirement.

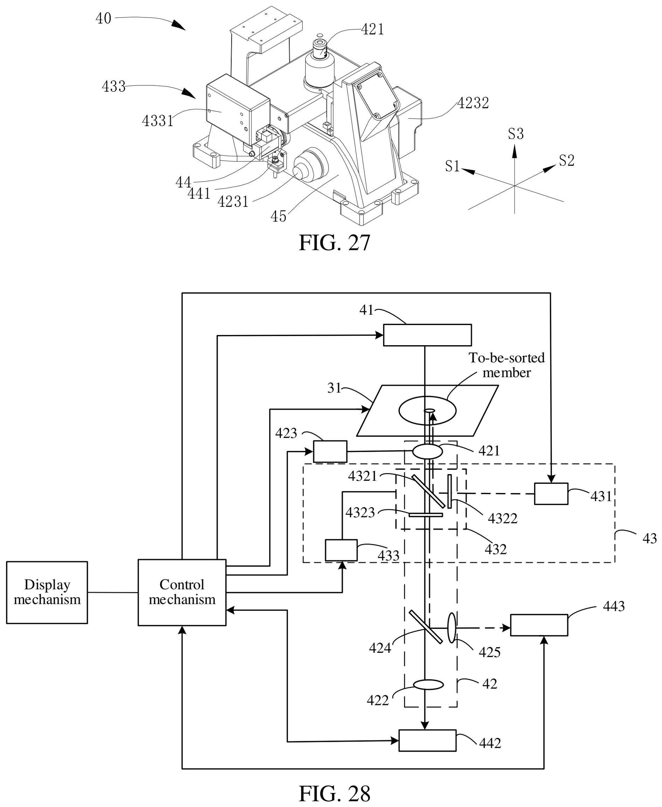

In some embodiments, referring to FIG. 27 and FIG. 28, an imaging mechanism is shown according to some embodiments of this disclosure. The imaging mechanism 40 is configured to image the to-be-sorted cells and obtain image information of the to-be-sorted cells, to select a cell meeting a preset requirement. The imaging mechanism 40 includes an imaging lighting unit 41, an optical path assembly 42, a fluorescent assembly 43, and a detection assembly 44.

The imaging lighting unit 41 provides a bright field lighting light source for a to-be-sorted member and the optical path assembly 42, to irradiate the to-be-sorted cells with transmitted light, the transmitted light passes through the optical path assembly 42 to reach the detection assembly 44, and image information of the to-be-sorted cells is obtained through the detection assembly 44. With reference to FIG. 2 and FIG. 9, the imaging lighting unit 41 is spaced apart from the optical path assembly 42 in the third direction S3, and the to-be-sorted member is arranged between the imaging lighting unit 41 and the optical path assembly 42 in the third direction S3, so that the transmitted light emitted by the imaging lighting unit 41 enters the optical path assembly 42 after passing through the to-be-sorted member, thereby imaging the to-be-sorted cells.

Referring to FIG. 8 and FIG. 9 again, the imaging lighting unit 41 is configured to illuminate the to-be-sorted cells, to provide certain observation brightness for the to-be-sorted cells. The imaging lighting unit 41 is connected to the mounting assembly 22 in the third direction S3. With reference to FIG. 2 and FIG. 9, the imaging lighting unit 41 is connected to the accommodation member 223 in the third direction S3. The imaging lighting unit 41 is closer to the suck and release opening 212 of the sucking member 21 than the picking lighting unit 24 is. The imaging lighting unit 41 and the picking lighting unit 24 are spaced apart from each other in the axial direction of the sucking member 21 and the third direction S3.

In some embodiments, the imaging lighting unit 41 includes an imaging light-emitting source 411, a light shielding member 412, and a light transmitting member 413. Light emitted by the imaging light-emitting source 411 passes through the light shielding member 412 and the light transmitting member 413 to reach the to-be-sorted cells, and illuminates the to-be-sorted cells along the periphery of the sucking member 21. The imaging light-emitting source 411 is connected to the mounting assembly 22 in the third direction S3, so that light can be transmitted to the imaging mechanism 40, to meet a lighting requirement of the imaging mechanism 40. It should be noted that, a position of the imaging light-emitting source 411 is set according to an actual situation. The imaging light-emitting source 411 is provided with a central hole for the sucking member 21 to thread, so that the sucking member 21 can pass through the imaging light-emitting source 411. Further, with reference to FIG. 12 and FIG. 13, the first clamping driving member 2212 is connected to the imaging light-emitting source 411 through the central hole, and a diameter of the central hole is slightly greater than an outer diameter of the second clamping driving portion 2212c of the first clamping driving member 2212. A central axis of the imaging light-emitting source 411 in the third direction S3 coincides with or is parallel to a central axis of the sucking member 21 in the third direction S3, to facilitate lighting of the imaging light-emitting source 411.

In some embodiments, the imaging light-emitting source 411 is an annular light source, and provides bright and uniform lighting for a large field of view. Further, the imaging light-emitting source 411 is an LED light source. The annular LED light source may be an entire annular light source, or may be obtained by evenly spacing a plurality of light sources apart circumferentially, so that the imaging light-emitting source 411 irradiates the region of the to-be-sorted cells from the radial periphery of the sucking member 21. It should be noted that, the type of the annular light source is not limited to an LED light source.

Further, in some embodiments, referring to FIG. 9, the imaging light-emitting source 411 and the accommodation member 223 are in a detachable connection to each other. The detachable connection facilitates mounting and replacement of the sucking member 21, to prevent the sucking member 21 from touching the imaging light-emitting source 411 during mounting and replacement to damage the sucking member 21; and also facilitates mounting and replacement of the imaging light-emitting source 411.

In some embodiments, during use of the cell sorting apparatus 100, the sucking member 21 is first mounted to the clamping unit 221, the clamping unit 221 is then placed in the accommodation member 223, and finally the imaging lighting unit 41 passes through the sucking member 21 in the third direction S3 and is connected to the accommodation member 223, to prevent the sucking member 21 from touching the imaging lighting unit 41 during mounting to damage the sucking member 21.

Specifically, in an embodiment, the imaging light-emitting source 411 and the accommodation member 223 are magnetically connected to each other. In one of the embodiments, a magnet is arranged on an end surface of the imaging light-emitting source 411, the accommodation member 223 is made of a metal material, and a light source cover and the accommodation member 223 are magnetically connected by attracting the metal material through the magnet. In a feasible embodiment, the accommodation member 223 is made of a non-metal material, a magnetic metal member is arranged on the accommodation member 223, and a magnetic connection between the light source cover and the accommodation member 223 is implemented by attracting the magnetic metal member through the magnet. It may be understood that positions of the magnet and the magnetic metal member are interchangeable, and specific arrangement positions are not limited. For example, the light source cover is provided with the magnetic metal member, and the accommodation member 223 is provided with the magnet, to implement the connection between the light source cover and the accommodation member 223. The magnetic connection facilitates the detachable connection between the mounting assembly 22 and the lighting assembly 24, which facilitates mounting and replacement of the sucking member 21. In addition, the magnetic connection does not destroy structures of the mounting assembly 22 and the lighting assembly 24, is reliable, stable, convenient, and fast, and does not require precise alignment.

In an embodiment not shown in the figure, the imaging light-emitting source 411 is provided with a groove, and the accommodation member 223 is provided with a protrusion for matching the groove. A detachable connection between the mounting assembly 22 and the lighting assembly 24 is implemented by combining the groove and the protrusion. It may be understood that, as long as the connection between the imaging light-emitting source 411 and the accommodation member 223 can be implemented, the connection manner between the imaging light-emitting source 411 and the accommodation member 223 is not limited to a detachable connection. For example, the imaging light-emitting source 411 is glued to the accommodation member 223.

In some embodiments, the light transmitting member 413 and the light shielding member 412 jointly limit scattering of light of the imaging light-emitting source 411. The light shielding member 412 is arranged between the imaging light-emitting source 411 and the light transmitting member 413 in the third direction S3. The light shielding member 412 is in the shape of a hollow cylinder as a whole, and extends in the third direction S3, to thread the sucking member 21. An outer diameter of the light shielding member 412 matches an outer diameter of the imaging light-emitting source 411. The light transmitting member 413 is a transparent circular ring as a whole and is provided with a central hole. An outer diameter of the light transmitting member 413 matches the outer diameter of the imaging light-emitting source 411, and a shape and a size of the central hole match an outer diameter shape and a size of the sucking member 21, to thread the sucking member 21. The light transmitting member 413 may be an acrylic sheet. It may be understood that the shapes, sizes, and materials of the light shielding member 412 and the light transmitting member 413 are not limited as long as light scattering and light transmission can be limited.

In some embodiments, the light transmitting member 413, the light shielding member 412, and the imaging light-emitting source 411 are in a detachable connection to each other. The detachable connection facilitates maintenance and replacement. Specifically, the light transmitting member 413, the light shielding member 412, and the imaging light-emitting source 411 are each coaxially provided with a threaded hole in the third direction S3, and the light transmitting member 413, the opaque member 413, and the imaging light-emitting source 411 are connected to each other by threading a fastener through the threaded holes. It should be noted that, the connection between the imaging light-emitting source 411, the light shielding member 412, and the light transmitting member 413 is not limited to a detachable connection, and the connection between the imaging light-emitting source 411, the light shielding member 412, and the light transmitting member 413 may alternatively be implemented by gluing.

If the imaging light-emitting source 411 is a light source for transmitting light, the imaging light-emitting source 411 only needs to be a light source that emits light including a wavelength that can be absorbed or reflected by the to-be-sorted cells. Various lamps such as a halogen lamp, a light-emitting diode, and various lasers can be used as the imaging light-emitting source 411. An image from the to-be-sorted cells may be image information such as a bright field image or a dark field image. Further, in some embodiments, brightness of the imaging light-emitting source 411 may be adjusted, so that the imaging light-emitting source 411 can be applied to more scenarios and brightness adjustment of the image information is facilitated.

In some embodiments, referring to FIG. 28, the optical path assembly 42 is configured to form an optical path of the imaging mechanism 40, and is equipped with an optical element configured to form a transmission optical path, to image the to-be-sorted cells. The optical path assembly 42 includes an objective lens 421 and a first imaging lens 422. The objective lens 421 has a specific focal length, a preset region of the to-be-sorted member and the objective lens 421 are spaced apart from each other in the third direction S3, and the objective lens 421 images the to-be-sorted cells in the preset region. The objective lens 421 and the first imaging lens 422 are spaced apart in the third direction S3, to form a transmitted light optical path of the imaging mechanism 40. The transmitted light emitted by the imaging light-emitting source 411 sequentially passes through the to-be-sorted cells, the objective lens 421, and the first imaging lens 422 to reach the detection assembly 44. Specifically, the objective lens 421 may be a 10X objective lens 421. It should be noted that, the magnification of the objective lens 421 may be set according to actual needs, and is not limited herein. In another embodiment, the optical path assembly 42 may include a plurality of objective lenses 421 to obtain image information of the to-be-sorted cells under different magnifications. Because the objective lens 421 and the first imaging lens 422 are arranged on one side of the to-be-sorted cells in the third direction S3, light generated by the imaging light-emitting source 411 and light generated at the to-be-sorted cells may alternatively not be transmitted light but reflected light.

In some embodiments, referring to FIG. 27 and FIG. 28, the optical path assembly 42 further includes an objective lens movement unit 423. The objective lens movement unit 423 is configured to focus the objective lens 421, to change a focal length between the objective lens 421 and the to-be-sorted member, so that imaging of the to-be-sorted cells is clear, and the imaging mechanism 40 obtains clear image information of the to-be-sorted cells. The objective lens movement unit 423 can drive the objective lens 421 to move in the third direction S3 relative to the to-be-sorted member, so that image information of the to-be-sorted cells is clear.

In some embodiments, the objective lens movement unit 423 includes a first rotating member 4231 and a second rotating member that is not shown in the figure, and the first rotating member 4231 and the second rotating member are arranged opposite to each other in the second direction S2. Both the first rotating member 4231 and the second rotating member can move the objective lens 421 in the third direction S3, to focus the objective lens 421. The second rotating member can implement automatic movement of the objective lens 421 under the control of the control mechanism 60. The first rotating member 4231 is not controlled by the control mechanism 60, and can implement movement of the objective lens 421 under the action of an external force. The arrangement of the first rotating member 4231 and the second rotating member can enable the objective lens 421 to perform both automatic adjustment and focusing and manual adjustment and focusing, thereby improving applicability of the cell sorting apparatus 100.

In some embodiments, the objective lens movement unit 423 further includes an objective lens driving member 4232. The objective lens driving member 4232 is connected to the control mechanism 60 and can drive the second rotating member to rotate, to move the objective lens 421 in the third direction S3, and a distance between the objective lens 421 and the to-be-sorted cells is adjusted to focus the objective lens 421, thereby obtaining high-definition image information of the to-be-sorted cells. The objective lens driving member 4232 may be a stepper motor, to accurately control a movement distance of the objective lens 421, thereby implementing coarse adjustment and fine adjustment during focusing of the objective lens 421.

In some embodiments, the objective lens movement unit 423 further includes a displacement sensor that is not shown in the figure. The arrangement of the displacement sensor makes it convenient to control a displacement of the objective lens 421, to help automatic focusing of the objective lens 421. In this embodiment, the displacement sensor is a grating displacement sensor. It may be understood that a type of the displacement sensor is not limited as long as position information of the support assembly 31 can be obtained.

In some embodiments, the objective lens movement unit 423 further includes an objective lens limiting member not shown in the figure. The arrangement of the objective lens limiting member limits a movement distance of the objective lens 421, and a highest position and a lowest position of the objective lens 421 can be obtained in combination with the displacement sensor, to prevent the objective lens 421 from colliding with the support mechanism 30 to damage the objective lens 421, thereby facilitating automatic movement of the objective lens 421 to implement focusing. With reference to FIG. 19 to FIG. 22, the first support member 311 is arranged as a step-shaped structure in the third direction S3, to prevent the second support member 312 from colliding with the accommodation unit 314; and provide a focusing movement distance for the objective lens 421, and prevent the objective lens 421 from colliding with the first support member 311 during focusing to damage the objective lens 421. Further, a lens surface of the objective lens 421 is arranged between the first support member 311 and the second support member 312 in the third direction S3, to better focus the objective lens 421 and ensure clear image information of the to-be-sorted cells. The third support member 313 is provided with an observation hole 3132, to facilitate focusing of the objective lens 421 and prevent the objective lens 421 from colliding with the third support member 313. The second support member 312 is provided with a through-hole, to focus and thread the objective lens 421. A size of the through-hole should ensure that movement of the second support member 312 does not collide with the objective lens 421, thereby ensuring normal use of the cell sorting apparatus 100.

With reference to FIG. 2, in some embodiments, the objective lens 421 coincides with or is parallel to a central axis of the sucking member 21 in the third direction S3. That the objective lens 421 coincides with or is parallel to the central axis of the sucking member 21 helps improve accuracy of the sucking member 21 in aspirating a cell meeting the preset requirement, thereby improving screening efficiency of the cell sorting apparatus 100.

Referring to FIG. 27 and FIG. 28 again, the fluorescent assembly 43 is configured to enable the to-be-sorted cells to emit fluorescence, and find out a cell meeting the preset requirement through fluorescence image information of the to-be-sorted cells. The fluorescent assembly 43 and the optical path assembly 42 are arranged on the same side of the to-be-sorted cells. The fluorescent assembly 43 includes an excitation light source 431 and a filter unit 432. The excitation light source 431 is configured to illuminate the to-be-sorted cells and enable the to-be-sorted cells to emit fluorescence. The excitation light source 431 may be any light source that enables the to-be-sorted cells to emit fluorescence. A lamp such as a xenon lamp or a mercury lamp, various lasers such as an argon laser, and a light-emitting diode can be used as the excitation light source 431. The filter unit 432 is a core component of the imaging mechanism 40 to implement fluorescence imaging. In some embodiments, the filter unit 432 is arranged between the objective lens 421 and the first imaging lens 422 in the third direction S3, and includes a first beam splitter 4321. The first beam splitter 4321 can reflect light emitted by the excitation light source 431, and can transmit transmitted light and fluorescence that pass through the to-be-sorted cells, so that the detection assembly 44 obtains image information of the to-be-sorted cells. The first beam splitter 4321 can split a wavelength of the imaging light-emitting source 411 from a wavelength of fluorescence generated by the to-be-sorted cells, and is, for example, a dichroic mirror or a dichromatic mirror.

The first beam splitter 4321 is arranged between the objective lens 421 and the first imaging lens 422 in the third direction S3. The first beam splitter 4321 is arranged at an angle to the third direction S3. The first beam splitter 4321 is spaced apart from the excitation light source 431 in the second direction S2, to reflect light emitted by the excitation light source 431 to the to-be-sorted cells, thereby enabling the to-be-sorted cells to emit fluorescence.

In some embodiments, the filter unit 432 further includes a first filtering member 4322. The first filtering member 4322 is arranged between the first beam splitter 4321 and the excitation light source 431 in the second direction S2. The first filtering member 4322 is configured to filter the excitation light source 431, to cause light in a wavelength band enabling the to-be-sorted cells to emit fluorescence to reach the first beam splitter 4321.

The observation optical path in the imaging mechanism 40 includes a lighting optical path obtained when the light emitted by the imaging light-emitting source 411 passes through the to-be-sorted cells, a guide optical path for guiding the light emitted by the excitation light source 431 to the to-be-sorted cells, and a fluorescence optical path for generating fluorescence by the to-be-sorted cells.

In some embodiments, the fluorescent assembly 43 further includes a filtering switching unit 433. The filtering switching unit 433 is configured to switch different filter units 432, and excitation light in different wavelength bands is obtained through the different filter units 432, to enable the to-be-sorted cells to emit different fluorescence, so that the imaging mechanism 40 can meet culture requirements of the to-be-sorted cells in a plurality of cases, thereby improving applicability of the cell sorting apparatus 100.

In some embodiments, the to-be-sorted cells undergo specificity screening by using a culture medium with a preset type of fluorescence dye added. Correspondingly, different fluorescence dyes may generate different fluorescence under the action of excitation light. For example, the to-be-sorted cells are cultured by using three fluorescence dyes. After the excitation light passes through a first filter unit 432, the to-be-sorted cells are enabled to emit first fluorescence. After the excitation light passes through a second filter unit 432, the to-be-sorted cells are enabled to emit second fluorescence. After the excitation light passes through a third filter unit 432, the to-be-sorted cells are enabled to emit third fluorescence.

Specifically, in an embodiment, the to-be-sorted cells undergo specificity screening by adding a culture medium containing a fluorescence dye capable of generating blue fluorescence, a fluorescence dye capable of generating green fluorescence, and a fluorescence dye capable of generating red fluorescence. Light with a short wavelength is excited to generate light with a long wavelength. Therefore, ultraviolet is excited to generate blue light, blue light is excited to generate green light, and green light is excited to generate red light. An ultraviolet filter unit 432 is selected for the to-be-sorted cells cultured by using the fluorescence dye capable of generating blue fluorescence, and excitation light enables the to-be-sorted cells to emit blue fluorescence. A blue light filter unit 432 is selected for the to-be-sorted cells cultured by using the fluorescence dye capable of generating green fluorescence, and excitation light enables the to-be-sorted cells to emit green fluorescence. A green light filter unit 432 is selected for the to-be-sorted cells cultured by using the fluorescence dye capable of generating red fluorescence, and excitation light enables the to-be-sorted cells to emit red fluorescence. The to-be-sorted cells undergo specificity screening by the preset type of fluorescence dye, which helps to more accurately select a cell meeting the preset requirement. It may be understood that different filter units 432 are selected according to different fluorescence dyes, and types of the fluorescence dyes and the filter units 432 are not limited.

In some embodiments, the filtering switching unit 433 includes a switching driving member 4331. The switching driving member 4331 is connected to the filter unit 432, and can drive the filter unit 432 to move, to switch different filter units 432. The switching driving member 4331 is arranged on a side of the objective lens 421 in the second direction S2, and each filter unit 432 connected to the switching driving member 4331 can be moved to the observation optical path of the imaging mechanism 40, for example, moved to a position between the objective lens 421 and the first imaging lens 422 in the third direction S3. With reference to FIG. 2, the control mechanism 60 can control the filtering switching unit 433 to switch different filter units 432, to excite different fluorescence dyes to generate different fluorescence.

In some embodiments, the filter unit 432 is equipped with a first filtering member 4322, a second filtering member, and a third filtering member. The first filtering member 4322, the second filtering member, and the third filtering member are different from each other. The filtering switching unit 433 switches the first filtering member 4322, the second filtering member, and the third filtering member. When the filtering switching unit 433 switches to the first filtering member 4322, the light emitted by the excitation light source 431 passes through the first filtering member 4322 to enable the to-be-sorted cells to emit first fluorescence. When the filtering switching unit 433 switches to the second filtering member, the light emitted by the excitation light source 431 passes through the second filtering member to enable the to-be-sorted cells to emit second fluorescence. When the filtering switching unit 433 switches to the third filtering member, the light emitted by the excitation light source 431 passes through the third filtering member to enable the to-be-sorted cells to emit third fluorescence. It should be noted that, the quantity of filtering members may be set according to the required fluorescence emitted by the to-be-sorted cells.

In some embodiments, the filtering switching unit 433 includes a switching driving member 4331 connected to the control mechanism 60. The switching driving member 4331 is connected to the excitation light source 431, so that the to-be-sorted cells are enabled to emit different fluorescence by switching the excitation light source 431. For example, three groups of excitation light sources 431 that can excite different fluorescence and that enter the observation optical path may be switched, and excitation light with different wavelengths is emitted by different excitation light sources 431 such as an ultraviolet LED light source, a blue LED light source, and a green LED light source, to enable the to-be-sorted cells to emit different fluorescence. It should be noted that, the filtering switching unit 433 may alternatively automatically switch different light source switching in-out optical paths, switch different filter units 432, or switch different filtering member in-out optical paths in a manual manner. A specific setting manner may be set according to actual needs, and a switching manner of the excitation light sources 431 is not limited herein.

In some embodiments, the filter unit 432 further includes a fourth filtering member 4323. The fourth filtering member 4323 is arranged between the first beam splitter 4321 and the first imaging lens 422 in the third direction S3. The fourth filtering member 4323 is configured to filter fluorescence emitted by the to-be-sorted cells, so that fluorescence image information of the to-be-sorted cells reaching the detection assembly 44 is not affected by other light.

The filter unit 432 plays an essential role in the fluorescence imaging of the imaging mechanism 40: After the light emitted from the excitation light sources 431 is emitted to the first filtering member 4322, the first filtering member 4322 selects an excitation light source 431 in a certain wavelength band to excite a sample, and blocks light of other wavelengths; and the light passing through the first filtering member 4322 passes through the first beam splitter 4321, the first beam splitter 4321 reflects the filtered excitation light, the reflected filtered excitation light is focused by the objective lens 421 and is irradiated to the to-be-sorted cells, to enable the to-be-sorted cells to emit corresponding fluorescence, and the fluorescence is collected by the objective lens 421, passes through the first beam splitter 4321, and reaches the fourth filtering member 4323. When the first beam splitter 4321 is inclined at 45 degrees toward the excitation light source 431, the filtered excitation light is emitted perpendicularly to the objective lens 421. In this case, the objective lens 421 directly functions as a condenser. In a feasible embodiment, the first filtering member 4322, the fourth filtering member 4323, and the first beam splitter 4321 are integrally arranged, to obtain the filter unit 432.

Referring to FIG. 28, the detection assembly 44 is connected to a display mechanism 50 for image observation and processing. The detection assembly 44 is configured to detect image information formed by transmitted light and fluorescence of the to-be-sorted cells emitted by the optical path assembly 42, process the obtained image information, and transmit the obtained image information to the display mechanism 50. In an embodiment, the detection assembly 44 includes a CCD sensor 441. In another embodiment, the detection assembly 44 includes a CMOS sensor 441. Either the CMOS sensor 441 or the CCD sensor 441 can obtain bright field image information and fluorescence image information of the to-be-sorted cells, to facilitate subsequent processing and screening. Further, the detection assembly 44 includes a cooling unit, which is not shown in the figure. The CMOS sensor 441 or the CCD sensor 441 is cooled by using the cooling unit such as liquid nitrogen or a Peltier element, to improve a signal-to-noise ratio and reduce noise of image information.

In some embodiments, the detection assembly 44 includes a bright field image information detector 442 and a fluorescence image information detector 443. The bright field image information detector 442 is configured to detect image information of transmitted light and reflected light passing through the to-be-sorted cells based on light of the imaging light-emitting source 411. The fluorescence image information detector 443 is configured to detect image information of fluorescence emitted by the to-be-sorted cells, and both the bright field image information detector 442 and the fluorescence image information detector 443 are connected to the CMOS sensor 441 or the CCD sensor 441.

In some embodiments, the optical path assembly 42 is provided with a second beam splitter 424 and a second imaging lens 425. The second beam splitter 424 is spaced apart from the first beam splitter 4321 in the third direction S3, and is arranged between the first beam splitter 4321 and the first imaging lens 422. The transmitted light that comes from the microscopic cells and that passes through the first beam splitter 4321 passes through the second beam splitter 424 and the first imaging lens 422, and is emitted to the bright field image information detector 442. Further, a filter that is not shown in the figure is arranged between the first imaging lens 422 and the bright field image information detector 442, so that the transmitted light is incident onto the bright field image information detector 442 without interfering with the excitation light source 431 or the fluorescence.

The second imaging lens 425 is arranged between the second beam splitter 424 and the fluorescence image information detector 443. The fluorescence that comes from the to-be-sorted cells and that passes through the first beam splitter 4321 is reflected by the second beam splitter 424 and passes through the second imaging lens 425, and then is emitted to the fluorescence image information detector 443. Further, a filter that is not shown in the figure is arranged between the imaging lens and the fluorescence image information detector 443, so that the fluorescence is incident onto the fluorescence image information detector 443 without interfering with the excitation light source 431 or the transmitted light.

The first imaging lens 422 used by the bright field image information detector 442 and the second imaging lens 425 used by the fluorescence image information detector 443 may be the same lens or different lenses. For example, the magnification of the first imaging lens 422 used by the bright field image information detector 442 may be reduced, and meanwhile the magnification of the second imaging lens 425 used by the fluorescence image information detector 443 may be increased. In this case, the field of view of the image of the transmitted light obtained by the bright-field image information detector 442 can be enlarged, and a larger fluorescence image of the to-be-sorted cells can be obtained in the fluorescence image information detector 443.

In some embodiments, with reference to FIG. 2 and FIG. 27, the imaging mechanism 40 further includes a protective housing 45, configured to protect internal components of the imaging mechanism 40 and protect an optical path of the imaging mechanism 40 from external light, thereby ensuring accurate and clear image information of the to-be-sorted cells. The protective housing 45 is connected to the bottom plate 1211 through a fastener. Fixing bumps for fixing the protective housing 45 are further arranged on the bottom plate 1211, so that the protective housing 45 is limited by the plurality of fixing bumps, thereby ensuring stable placement of the imaging mechanism 40.

Before the imaging mechanism 40 is used, the imaging lighting unit 41, the to-be-sorted cells, and the objective lens 421 are arranged in the third direction S3. During use, bright field image information of the to-be-sorted cells is obtained, and specific operations are as follows: The imaging lighting unit 41 is turned on, so that light emitted by the imaging light-emitting source 411 enters the bright field image information detector 442 through the to-be-sorted cells, the objective lens 421, the filter unit 432, and the first imaging lens 422, and the bright field image information detector 442 transmits the obtained image information of the to-be-sorted cells to the display mechanism 50. The objective lens movement unit 423 is controlled, according to the image information displayed by the display mechanism 50, to move the objective lens 421 in the third direction S3, so that the image information of to-be-sorted cells is clear and recorded by the CMOS sensor 441 or the CMOS sensor, to facilitate subsequent processing.

Fluorescence image information of the to-be-sorted cells is obtained, and specific operations are as follows: The imaging light-emitting source 411 is turned off, the excitation light source 431 is turned on, and light emitted by the excitation light source 431 passes through the filter unit 432 and the objective lens 421 to reach the to-be-sorted cells, so that the to-be-sorted cells undergoing specificity screening through the fluorescence dye emit fluorescence, the fluorescence enters the fluorescence image information detector 443 through the objective lens 421, the filter unit 432, and the second imaging lens 425, and the fluorescence image information detector 443 transmits fluorescence image information of the to-be-sorted cells to the display mechanism 50. Based on the image information obtained by the display mechanism 50, the objective lens movement unit 423 is controlled to move a position of the objective lens 421, so that the image information is clear, and then the image information is recorded by using the CCD sensor 441 or the CMOS sensor. A replacement with a different filter unit 432 is made by the filtering switching unit 433, and the foregoing operation is repeated, so that different fluorescence image information of the to-be-sorted cells is transmitted to the display mechanism 50. A cell meeting the preset requirement is found by analyzing the obtained bright field image information and fluorescence image information of the to-be-sorted cells.

It should be noted that, switching between the imaging light-emitting source 411 and the excitation light source 431 may be implemented through the control mechanism 60, or may be implemented through manual switching.

The to-be-sorted member can automatically move under the control of the control mechanism 60. The fluorescent assembly 43 is provided with different filter units 432. Automatic switching is implemented by the filtering switching unit 433, and automatic focusing is implemented by the objective lens movement unit 423, to scan the imaging mechanism 40 automatically. Automatic scanning and imaging can be performed on a large quantity of to-be-sorted cells, thereby significantly improving the working efficiency of the cell sorting apparatus 100 and expanding the disclosure range thereof. In addition, through contrastive analysis of a plurality of types of fluorescence image information and bright field image information before and after selection, cell sorting is more accurately implemented.

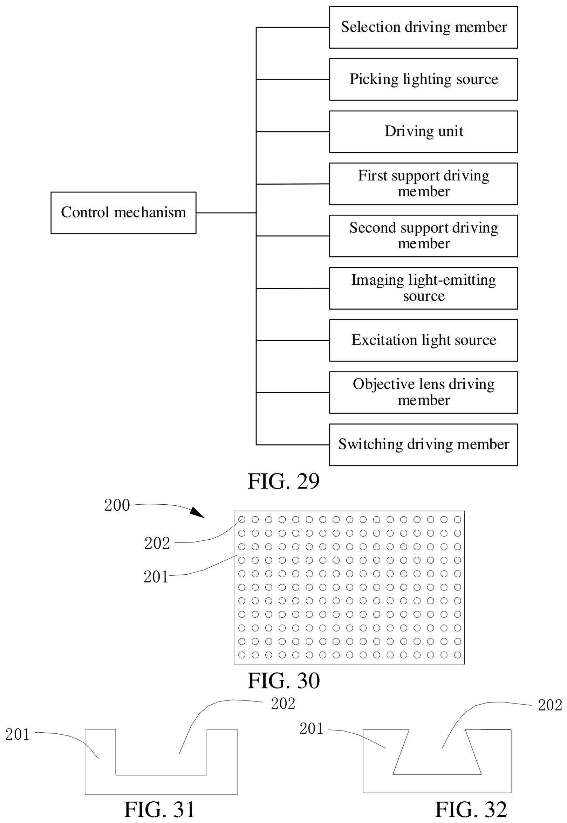

Referring to FIG. 29, in some embodiments, the control mechanism 60 is configured to control the cell sorting apparatus 100, to implement automatic cell sorting, improve cell sorting efficiency, and implement high-throughput screening. The control mechanism 60 can control the selection driving member 231, to control movement and stop of the sucking member 21 in the third direction S3. The control mechanism 60 can control turn-on and turn-off of the picking lighting source 241, to observe and align the sucking member 21. The control mechanism 60 can control the driving unit 251, to clean the sucking member 21 and control the sucking member 21 to suck or release a cell meeting the preset requirement. The control mechanism 60 can control the first support driving member 3211 and the second support driving member 3221, to control movement and stop of the support assembly 31. The control mechanism 60 can control turn-on and turn-off of the imaging light-emitting source 411, to provide a transmitted light source for the to-be-sorted cells, and obtain bright field image information of the to-be-sorted cells. The control mechanism 60 can control turn-on and turn-off of the excitation light source 431, to provide the excitation light source 431 for the to-be-sorted cells, and obtain fluorescence image information of the to-be-sorted cells. The control mechanism 60 can control the objective lens driving member 4232, to focus the objective lens 421, and obtain clear image information of the to-be-sorted cells. The control mechanism 60 can control the switching driving member 4331, to excite the to-be-sorted cells to emit different fluorescence.

In some embodiments, the control mechanism 60 is arranged on the body 10, and includes a power supply module, a turn-on/turn-off module, a switching module, a voltage module, and the like that are not shown in the figure. It may be understood that, as long as the control of each functional component of the cell sorting apparatus 100 belongs to the control mechanism 60, a corresponding control element may be selected for the control mechanism 60 according to the arrangement of the components of the cell sorting apparatus 100.

In some embodiments, the cell sorting apparatus 100 further includes a lighting assembly. The lighting assembly is configured to illuminate the hollow pipe 211 of the sucking member 21, and further configured to illuminate the imaging mechanism 40, thereby observing and aligning the sucking member 21 on the display mechanism 50. The lighting assembly includes one or both of the picking lighting unit 24 and the imaging lighting unit 41.

In some embodiments, the operation method of the cell sorting apparatus 100 includes the following steps:
The sucking member 21 is mounted to the mounting assembly 22. Specifically, first, the first housing 111 is moved to open the cell sorting apparatus 100. The first fixing member 2221 and the second fixing member 2222 are loosened, the clamping unit 221 is removed, the first clamping driving member 2212 is loosened, and the first clamping driving member 2212 should not be wholly separated from the clamping body 2211. An end of the sucking member 21 arranged opposite to the suck and release opening 212 is placed in the clamping unit 221, the first clamping driving member 2212 is tightened to the clamping mark 2218, and strong tightening that causes damage to the sucking member 21 should not be performed. The clamping unit 221 to which the sucking member 21 is fixed is placed back in the accommodation member 223. The first fixing member 2221 and the second fixing member 2222 are tightened to fix the clamping unit 221, thereby completing the mounting of the sucking member 21 to the mounting assembly 22.

A position of the sucking member 21 is calibrated. Specifically, under the control of the control mechanism 60, the picking lighting source 241 is turned on, and the picking movement assembly 23 drives the sucking member 21 to move in the third direction S3, until image information of a sucking member projection is clearly presented on the display mechanism 50. The sucking member 21 is fine-adjusted by using the first fixing member 2221 and the second fixing member 2222, so that a projection mark is in a preset position of a sucking member projection mapping. Then, the to-be-sorted member on which no to-be-sorted cell is arranged is placed in the fixing groove of the first support member 311. The picking movement assembly 23 drives the sucking member 21 to move in the third direction S3, until image information of the suck and release opening 212 is clearly presented on the display mechanism 50. Position information of the sucking member 21 is recorded in this case, to complete the position calibration of the sucking member 21.

The sucking member 21 is cleaned for the first time. Specifically, first, the first holding member 2551 is taken, a proper amount of first cleaning liquid 2541 is added, and the first holding member 2551 with the first cleaning liquid 2541 added is placed on the first support member 311, so that the first holding member 2551 and the sucking member 21 are spaced apart in the third direction S3. Under the control of the control mechanism 60, the selection driving member 231 drives the suck and release opening 212 of the sucking member 21 to move into the first cleaning liquid 2541, and the driving unit 251 drives the sucking member 21 to suck the first cleaning liquid 2541 into the hollow pipe 211 of the sucking member 21 to perform cleaning, and then drives the sucking member 21 to release the first cleaning liquid 2541 into the first holding member 2551. This is repeated a plurality of times to complete the first step of cleaning for a first preset number of times. Then, the second holding member 2552 is taken, a proper amount of second cleaning liquid 2542 is added, the second holding member 2552 with the second cleaning liquid 2542 added is placed on the first support member 311, the second holding member 2552 and the sucking member 21 are spaced apart from each other in the third direction S3, the selection driving member 231 drives the sucking member 21 to move into the second cleaning liquid 2542, and the driving unit 251 drives the sucking member 21 to suck the second cleaning liquid 2542 into the hollow pipe 211 of the sucking member 21 to perform cleaning, and then drives the sucking member 21 to release the second cleaning liquid 2542 into the second holding member 2552. This is repeated a plurality of times to complete the second step of cleaning for a second preset number of times. The to-be-sorted member and the accommodation unit 314 are placed. The to-be-sorted member on which the to-be-sorted cells are arranged is placed in the support portion 3111 of the first support member 311, and the elastic member 331 on one side of the support portion 3111 is pulled to the other side of the support portion 3111, so that the elastic member 331 eccentrically presses the to-be-sorted member, thereby placing and fixing the to-be-sorted member on the first support member 311. The accommodation unit 314 provided with a proper amount of culture solution is placed in the placement groove 3112 of the first support member 311. The to-be-sorted cells undergo specificity screening through a culture medium containing a fluorescence dye.

A cell that meets the preset requirement is found. Under the control of the control mechanism 60, the imaging light-emitting source 411 is turned on, the first support movement unit 321 and the second support movement unit 322 drive the to-be-sorted cells that need to be observed in the to-be-sorted member, the second light-emitting source, and the objective lens 421 to be arranged in the third direction S3, the objective lens moving unit 423 drives the objective lens 421 to move until image information of the to-be-sorted cells that need to be observed is clearly presented on the display mechanism 50, and bright field image information of the to-be-sorted cells is recorded. The imaging light-emitting source 411 is turned off, the excitation light source 431 is turned on, to cause light emitted by the excitation light source 431 to pass through the first filter unit 432 and cause the to-be-sorted cells to emit first fluorescence, and first fluorescence image information of the to-be-sorted cells is recorded. The filtering switching unit 433 is controlled to switch to the second filter unit 432, to cause the to-be-sorted cells to emit second fluorescence, and second fluorescence image information of the to-be-sorted cells is recorded. The filtering switching unit 433 is controlled to switch to the third filter unit 432, to cause the to-be-sorted cells to emit third fluorescence, and third fluorescence image information of the to-be-sorted cells is recorded. Then, the excitation light source 431 is turned off, and the imaging light-emitting source 411 is turned on. The first support movement unit 321 and the second support movement unit 322 drive the to-be-sorted member to move to a preset region, to replace the to-be-sorted cells that need to be observed. The foregoing process is repeated to record bright field image information and fluorescence image information of the to-be-sorted cells in different regions. The obtained bright field image information and fluorescence image information of the to-be-sorted cells are processed, and analyzed to find a cell meeting the preset requirement.

A cell meeting the preset requirement is sucked and recovered. Under the control of the control mechanism 60, the picking movement assembly 23 drives the sucking member 21 to move in the third direction S3 until the sucking member 21 reaches a position in which cells can be sucked, where the position is marked as the preset position, and the pre-suck is completed. The second support movement unit 322 drives the second support member 312 to move in the second direction S2, and the first support movement unit 321 drives the first support member 311 to move in the first direction S1, until the cell meeting the preset requirement and the sucking member 21 are spaced apart in the third direction S3. The picking movement assembly 23 drives the sucking member 21 to move to the preset position in the third direction S3. The driving unit 251 causes an appropriate negative pressure to be generated in the hollow pipe 211 of the sucking member 21, to suck cells into the hollow pipe 211 of the sucking member 21. The picking movement assembly 23 drives the sucking member 21 to move in a direction away from the support assembly 31. The second support movement unit 322 drives the second support member 312 to move in the second direction S2, and the first support movement unit 321 drives the first support member 311 to move in the first direction S1, until the holding portion 3142a for recovering and culturing a cell meeting the preset requirement and the sucking member 21 are spaced apart in the third direction S3. The picking movement assembly 23 drives the sucking member 21 to move in the third direction S3, so that there is a preset distance between the suck and release opening 212 and the receiving portion 3142a. The preset distance is a distance enabling the sucking member 21 to release a cell meeting the preset requirement to the receiving portion 3142a. The driving unit 251 drives the sucking member 21 to release, into the accommodation portion 3142a, the cell that meets the preset requirement and that is sucked in the hollow pipe 211. The picking movement assembly 23 drives the sucking member 21 to move in a direction away from the support assembly 31. The foregoing process is repeated, so that different a cell meeting the preset requirement are sucked, released, and recovered into different accommodation portions 3142a, until all the cell meeting the preset requirement in the to-be-sorted member are sucked and released. The to-be-sorted member is recovered and preserved, and the accommodation unit 314 is transferred to the incubator to continue to be cultured.

The sucking member 21 is cleaned for the second time. Specifically, first, the second holding member 2552 is taken, a proper amount of second cleaning liquid 2542 is added, the second holding member 2552 with the second cleaning liquid 2542 added is placed on the first support member 311, the second holding member 2552 and the sucking member 21 are spaced apart from each other in the third direction S3, the selection driving member 231 drives the sucking member 21 to move into the second cleaning liquid 2542, and the driving unit 251 drives the sucking member 21 to suck the second cleaning liquid 2542 into the hollow pipe 211 of the sucking member 21 to perform cleaning, and then drives the sucking member 21 to release the second cleaning liquid 2542 into the second holding member 2552. This is repeated a plurality of times to complete the first step of cleaning for a preset number of times. Then, the first holding member 2551 is taken, a proper amount of first cleaning liquid 2541 is added, and the first holding member 2551 with the first cleaning liquid 2541 added is placed on the first support member 311, so that the first holding member 2551 and the sucking member 21 are spaced apart in the third direction S3. Under the control of the control mechanism 60, the selection driving member 231 drives the suck and release opening 212 of the sucking member 21 to move into the first cleaning liquid 2541, and the driving unit 251 drives the sucking member 21 to suck the first cleaning liquid 2541 into the hollow pipe 211 of the sucking member 21 to perform cleaning, and then drives the sucking member 21 to release the first cleaning liquid 2541 into the first holding member 2551. This is repeated a plurality of times to complete the second step of cleaning for a preset number of times.

Further, the operation method of the cell sorting apparatus 100 further includes:
Obtaining bright field image information of the sucked to-be-sorted cells. Specifically, before the sucking member 21 is cleaned for the second time, and after the cell meeting the preset requirement are sucked and recovered, under the control of the control mechanism 60, the imaging light-emitting source 411 is turned on, the first support movement unit 321 and the second support movement unit 322 drive the to-be-sorted cells that need to be observed in the to-be-sorted member, the second light-emitting source, and the objective lens 421 to be arranged in the third direction S3, the objective lens moving unit 423 drives the objective lens 421 to move until image information of the to-be-sorted cells that need to be observed is clearly presented on the display mechanism 50, and bright field image information of the to-be-sorted cells is recorded. The first support movement unit 321 and the second support movement unit 322 drive the to-be-sorted member to move to a preset region, to replace the to-be-sorted cells that need to be observed. The foregoing process is repeated until all bright field image information of the to-be-sorted cells in different regions is obtained and recorded. Bright field image information of the to-be-sorted cells before and after the suck is compared, to get an obtaining status of a cell meeting the preset requirement.

A cell meeting the preset requirement in the to-be-sorted member are selectively sucked and released into a corresponding accommodation portion 3142a of the accommodation unit 314 by using the cell sorting apparatus 100. This process is automatically completed under the control of the control mechanism 60, thereby improving the efficiency of cell sorting, avoiding damage caused by a manual operation to the sucking member 21 and the cell meeting the preset requirement, ensuring biological activity of the cell meeting the preset requirement, and implementing high-throughput cell sorting.

The biological culture chip 200 makes full use of a microscopic technology or a nanotechnology to complete accurate control of cells such as capture, fixation, and culture on the chip, and achieves research objectives such as high-throughput, multi-parameter, and in-situ signal detection of a cell sample, and physical-chemical analysis of cell components by using a miniaturized chemical analysis method.

The biological culture chip 200 is described in detail below.

FIG. 30 to FIG. 32 are schematic diagrams of a biological culture chip according to some embodiments of this disclosure.

In some embodiments, referring to FIG. 30 to FIG. 32, a biological culture chip 200 includes a matrix 201 and an accommodation cavity 202, where the matrix 201 is made of a matrix material, the accommodation cavity 202 is formed on a surface of the matrix 201, and the accommodation cavity 202 opens on the surface of the matrix 201. The accommodation cavity 202 defines a biological culture space for culturing a cellular biological material. The structure and the size of the accommodation cavity 202 are not particularly limited, as long as a culture or culture and expansion space can be provided for a single cell. The accommodation cavity 202 may present a structure such as a cuboid, a cube, or a cylinder.

In some embodiments, the matrix material is a microporous soft matrix with good biocompatibility, cell culture medium compatibility, and mechanical tension, so that cells (including a single cell and a plurality of cells aggregated in a controllable quantity) can be captured and cultured more efficiently. The soft matrix is prone to affinity and fusion with different extra-cellular matrix environments, and provides a uniform and fused three-dimensional culture space for the cells, thereby creating, for the cells in a two-dimensional array, a microenvironment that is conducive to survival, proliferation, migration, protein expression and secretion, stem cell differentiation, and the like, and conducive to detection of biological functions of the cells in the array. It should be noted that a specific type of the matrix material that may be used is not particularly limited, and a hard matrix material such as glass, a silicon wafer, or plastic also falls within the protection scope of this disclosure.

In some embodiments, referring to FIG. 32, an opening diameter of the accommodation cavity 202 is less than a bottom diameter of the accommodation cavity 202. In this way, it may be convenient to capture a single cell for culture, and the cell does not escape during the culture. Specifically, the opening diameter of the accommodation cavity 202 is at most 80%, preferably 50%, of the bottom diameter of the accommodation cavity 202. The used term "diameter" is defined as a diameter of a maximum area circle that can be accommodated by the opening or the bottom. The used term "depth" of the accommodation cavity 202 refers to a shortest distance between an opening cross section and a bottom cross section of the accommodation cavity 202. In a feasible embodiment, the accommodation cavity 202 has an opening diameter ranging from 8 micrometers to 25 micrometers and a depth ranging from 15 micrometers to 35 micrometers. It should be noted that, different opening diameters may be set for different cells. For example, the biological material is a B-cell, and has an opening diameter ranging from 8 micrometers to 12 micrometers; and the biological material is a hybridoma cell or a tumor cell, and has an opening diameter ranging from 15 micrometers to 25 micrometers.

In some embodiments, the biological culture chip 200 may include a plurality of accommodation cavities 202, and the accommodation cavities 202 are distributed discretely. The accommodation cavities 202 form a predetermined pattern, and a distance between two adjacent accommodation cavities 202 ranges from 10 micrometers to 100 micrometers. In this way, the culture of a plurality of single cells can be processed in batches. When the distance between the accommodation cavities 202 ranges from 10 micrometers to 100 micrometers, cells cultured in the accommodation cavities 202 do not interfere with each other between the accommodation cavities 202. In this way, batch processing uniformity can be improved, accuracy and efficiency of detection of biological functions of the cells can be improved, and high-throughput collection and analysis of cell signal data are facilitated. The accommodation cavities 202 on the biological culture chip 200 may form an array, for example, an array of tens of thousands of accommodation cavities 202 or even hundreds of thousands of or more accommodation cavities 202 on one chip 200.

In some embodiments, positioning marks may be further arranged on the biological culture chip 200, and the positioning marks are formed on the matrix 201. Therefore, the plurality of accommodation cavities 202 may be positioned by using the positioning marks. The form of the positioning mark is not particularly limited. According to this embodiment of this disclosure, a positioning mark made of a fluorescence material may be arranged, so that automatic and accurate positioning can be performed, or a coordinate line may be arranged to position each accommodation cavity 202. After a cell meeting the preset requirement is observed, each accommodation cavity 202 in which a cell meeting the preset requirement is placed may be quickly positioned and recorded. In this way, the subsequent separation of the cell meeting the preset requirement or other components in the accommodation cavity 202 is further facilitated.

In some embodiments, a method for using the biological culture chip 200 is as follows:
The chip 200 is placed in a chip accommodation member 71. The biological culture chip 200 needs to be placed in a low-temperature environment for preservation, for example, placed in an environment of 0°C to 5°C. Therefore, before use, the biological culture chip 200 needs to stand at the room temperature for a period of time to have the temperature restored to the room temperature. The biological culture chip 200 is made of a thin, soft, and fragile material, and therefore is usually placed in the chip accommodation member 71. After being externally disinfected and sterilized, the chip accommodation member 71 in which the biological culture chip 200 is placed is transferred to an aseptic environment, and a chip preservation solution is taken out by using a pipette and cleaned for a plurality of times by using an aseptic PBS buffer solution with a concentration of 0.01 M. It should be noted that, the biological culture chip 200 is not touched during the cleaning.

Cells are added. The to-be-sorted cells prepared in advance are added to the chip accommodation member 71 accommodating the biological culture chip 200. Optionally, to ensure optimal single-cell array efficiency, a ratio of a quantity of cells to a throughput of the chip ranges from 5:1 to 10:1, and a volume of a cell suspension solution is 1.5 ml. For example, the throughput of the chip is 100,000, and a cell suspension solution containing cells whose number ranges from 500,000 to 1 million is arrayed. Stand in a cell incubator for a preset time, for example, stand for 15 minutes, so that the to-be-sorted cells form an array in the biological culture chip 200, and the accommodation cavities 202 of the biological culture chip 200 are arranged as an array.

Separation is performed for the first time. The arrayed cell suspension solution is recovered by using a pipette, then cleaned with a PBS buffer solution with a concentration of 0.01 M for a plurality of times to remove residual cells. The chip accommodation member 71 may be cleaned after being appropriately inclined. The entire process needs to be gently operated to separate cells that do not form an array. For the cleaned cell-arrayed chip 200, a state of the array is observed under a microscope.

A screening culture medium is prepared. A cell complete culture medium is prepared in advance. Each preset type of fluorescence dye is added to the cell complete culture medium according to a required dilution factor, shaken at a low rate, and fully mixed. A marker such as an antigen labeled with a fluorescence dye in advance is added to the thoroughly mixed cell complete culture medium according to a specific dilution ratio as required, shaken at a low rate, and thoroughly mixed, to obtain a screening culture medium.

The screening culture medium is added. The screening culture medium prepared in advance is added to the cell-arrayed chip 200, and then the cell-arrayed chip 200 is placed in a cell incubator for incubation. A substance in the screening culture medium and a secreted substance of the arrayed to-be-sorted cells undergo specificity binding. The incubation time is set according to different projects, for example, ranges from 3 hours to 8 hours for a cell line development project, and is not less than 8 hours for an antibody discovery project. In an antibody discovery project, an antigen labeled with a fluorescence dye in advance and added to a screening culture medium and an antibody secreted by a cell undergo specificity binding, and the antigen labeled with a fluorescence dye in advance is used as a marker. In a cell line development project, a second antibody that is labeled with a fluorescence dye in advance and that is added to a screening culture medium and an antibody secreted by a cell undergo specificity binding, and the second antibody that is labeled with a fluorescence dye in advance is used as a marker.

Separation is performed for the second time. After the incubation ends, the screening culture medium is recovered, and the chip is cleaned with a PBS buffer solution with a concentration of 0.01 M for 3 times to 5 times until all suspended cells are substantially cleared, and to-be-sorted cells cultured by the fluorescence dye are obtained from the biological culture chip 200.

FIG. 33 is a schematic diagram of a chip fixing assembly according to some embodiments of this disclosure. FIG. 34 is a schematic diagram of a pressure applying member according to some embodiments of this disclosure.

In some embodiments, referring to FIG. 19 and FIG. 33, the biological culture chip 200 is made of a thin, soft, and fragile material, and is difficult to fix in the cell sorting apparatus 100 directly. Therefore, the cell sorting apparatus 100 further includes a chip fixing assembly 70. The chip fixing assembly 70 can be placed on the support mechanism 30. The chip fixing assembly 70 includes a chip accommodation member 71, and the chip accommodation member 71 is configured to accommodate the biological culture chip 200. The biological culture chip 200 is first fixed in the chip accommodation member 71, and then the chip accommodation member 71 is placed on the support assembly 31 of the cell sorting apparatus 100.

In some embodiments, referring to FIG. 33, the chip fixing assembly 70 fixes the biological culture chip 200 into the chip accommodation member 71, and the chip fixing assembly 70 further includes a pressure applying member 72 supported by the chip accommodation member 71. The pressure applying member 72 can apply a pressure to the biological culture chip 200 in the third direction S3, so that the biological culture chip 200 is stably placed in the chip accommodation member 71. The pressure applying member 72 is in the shape of a cylinder with openings at two ends as a whole, and applies pressures to corners of the biological culture chip 200, so that the biological culture chip 200 is stably placed in the chip accommodation member 71. By applying the pressures to the corners of the biological culture chip 200, the quantity of effective accommodation cavities 202 in the biological culture chip 200 can be increased, and the quantity of to-be-sorted cells can be increased. It may be understood that, as long as the biological culture chip 200 can be fixed, a pressure applying position of the pressure applying member 72 is not limited, for example, the pressure applying member 72 applies pressures to edges of the biological culture chip 200. Further, to better fix the biological culture chip 200 in the chip accommodation member 71, the corners of the biological culture chip 200 abut against an inner wall of the chip accommodation member 71.

To facilitate placement and taking-out of the pressure applying member 72 and the biological culture chip 200, referring to FIG. 34, the pressure applying member 72 includes a pressure applying portion 722 and a taking-out portion 721. With reference to FIG. 33, the pressure applying portion 722 is accommodated in the chip accommodation member 71, and is configured to apply a pressure to the biological culture chip 200. The shape and the size of the pressure applying portion 722 match the shape and the size of the chip accommodation member 71. The taking-out portion 721 is configured to take out the pressure disclosure member 72, and is configured to place and take out the pressure disclosure member 72. An end of the pressure applying portion 722 away from the biological culture chip 200 extends in the circumferential direction to form the taking-out portion 721. The taking-out portion 721 protrudes from the chip accommodation member 71 in the circumferential direction of the chip accommodation member 71, that is, a size of at least part of the taking-out portion 721 is greater than a size of the chip accommodation member 71, to facilitate taking-out of the pressure applying member 72.

In some embodiments, the pressure applying member 72 may apply a pressure to the biological culture chip 200 through the body of the pressure applying member 72. Specifically, the pressure applying member 72 is made of a metal material such as copper or aluminum with a larger weight than that of the biological culture chip 200. A pressure is applied to the biological culture chip 200 through the gravity of the pressure applying member 72, and the mass of the pressure applying member 72 is set according to an actual requirement, and is not limited herein. The material of the pressure applying member 72 should ensure that the biological culture chip is not polluted, and the pressure applying member 72 needs to be disinfected and sterilized before and after use.

In some embodiments, the pressure applying member 72 may apply a pressure to the biological culture chip 200 by transferring an external pressure. Specifically, the pressure applying member 72 is made of a lightweight material, the pressure applying member 72 is provided with an engaging portion, and the chip accommodation member 71 is provided with an engaged portion. The pressure applying member 72 is connected to the chip accommodation member 71 through a combination of the engaging portion and the engaged portion, and the pressure exerted on the biological culture chip 200 is generated by the pressure applying member 72 through engagement, so that the biological culture chip 200 is fixed in the chip accommodation member 71. It may be understood that, as long as the biological culture chip 200 can be fixed in the chip accommodation member 71, a pressure disclosure manner of the pressure disclosure member 72 is not limited. In some embodiments, to ensure normal use of the biological culture chip 200 in the cell sorting apparatus 100, the cell sorting apparatus 100 further includes a fixing mechanism. The fixing mechanism includes a chip fixing assembly 70 and a support limiting assembly 33. The chip fixing assembly 70 is configured to fix the biological culture chip 200 in the chip accommodation member 71. The support limiting assembly 33 is configured to fix the chip accommodation member 71 on the support mechanism 30. The screening efficiency and accuracy of the cell sorting apparatus 100 are improved by using the biological culture chip 200.

In some embodiments, when the biological culture chip 200 undergoing cell specificity screening is used as the to-be-sorted member, the biological culture chip 200 is first fixed, the metal pressure applying member 72 is disinfected and sterilized, and the metal pressure applying member 72 is placed in the chip accommodation member 71, so that the biological culture chip 200 that is prepared in advance and in which the to-be-sorted cells are cultured is fixed in the chip accommodation member 71. Then, the chip accommodation member 71 is fixed, and the chip accommodation member 71 is placed in the support portion 3111 of the first support member 311. A through-hole and a limiting wall formed around the through-hole are formed on the support portion 3111. The elastic member 331 is arranged on an outer surface of the chip accommodation member 71. The elastic member 331 on one side of the support portion 3111 is pulled to the other side of the support portion 3111, so that the elastic member 331 eccentrically presses the outer surface of the chip accommodation member 71. The outer surface of the chip accommodation member 71 is eccentrically pressed and limited on the limiting wall, thereby fixing the chip accommodation member 71 on the first support member 311, and fixing the biological culture chip 200 on the cell sorting apparatus 100. Then, the foregoing operation method of the cell sorting apparatus 100 is repeated, to implement cell sorting.

In a feasible embodiment, the support limiting assembly further includes a limiting member 333 such as a silicone sleeve. The chip accommodation member 71 is placed in the limiting member 333, and then the limiting member 333 is placed in the support portion 3111. The elastic member 331 eccentrically presses an outer surface of the limiting member 333, to fix the chip accommodation member 71 on the first support member 311. It may be understood that the limiting member 333 may alternatively be first placed in the support portion 3111, and then the chip accommodation member 71 is placed in the limiting member 333.

During suck, the support movement assembly 32 drives the accommodation cavity 202 of the biological culture chip 200 on which the cell meeting the preset requirement are placed to move until the accommodation cavity 202 and the sucking member 21 are spaced apart in the third direction S3. The picking movement assembly 23 drives the sucking member 21 to move in the third direction S3 until there is a preset distance between the suck and release opening 212 and the accommodation cavity 202. The preset distance enables the driving unit 251 to drive the sucking member 21 to suck the cell meeting the preset requirement into the hollow pipe 211 of the sucking member 21. Optionally, the cell meeting the preset requirement is a single cell arranged in the accommodation cavity 202 of the biological culture chip 200.

The technical features in the foregoing embodiments may be randomly combined. For concise description, not all possible combinations of the technical features in the embodiment are described. However, provided that combinations of the technical features do not conflict with each other, the combinations of the technical features are considered as falling within the scope recorded in this specification.

The foregoing embodiments only describe several implementations of this disclosure specifically and in detail, but cannot be construed as a limitation to the patent scope of this disclosure. It should be noted that for a person of ordinary skill in the art, several transformations and improvements can be made without departing from the idea of this disclosure. These transformations and improvements belong to the protection scope of this disclosure. Therefore, the protection scope of the patent of this disclosure shall be subject to the appended claims.

## Claims

1. A cell sorting apparatus, comprising:
a body;
a support mechanism configured to be mounted on the body, wherein the support mechanism comprises a first support member configured to hold a to-be-sorted member for accommodating to-be-sorted cells, and the first support member is configured to move relative to the body, to cause the to-be-sorted member to move to a preset region relative to the body;
an imaging mechanism mounted on the body, configured to obtain image information of the to-be-sorted cells placed in the support mechanism, wherein the imaging mechanism comprises an objective lens configured to image the to-be-sorted cells in the preset region;
a display mechanism configured to display the image information of the to-be-sorted cells obtained by the imaging mechanism;
a picking mechanism mounted on the body, configured to suck a cell meeting a preset requirement in the to-be-sorted member, wherein the picking mechanism comprises a sucking member movable relative to the body is configured to suck and release the cell meeting the preset requirement; and
a control mechanism mounted on the body, configured to control movement of the support mechanism relative to the body, control movement of the sucking member relative to the body, and
control suction and release of the cell meeting the preset requirement by the sucking member.

2. The cell sorting apparatus according to claim 1, wherein the support mechanism comprises a support assembly and a support movement assembly, wherein the support assembly placed on the support movement assembly is configured to support the to-be-sorted member, and the support movement assembly is configured to drive the support assembly to move such that the to-be-sorted member is placed between the sucking member and the objective lens.

3. The cell sorting apparatus according to claim 2, wherein the support assembly comprises an accommodation unit placed on the support movement assembly, the accommodation unit configured to recover the cell meeting the preset requirement, and wherein, the support movement assembly is configured to drive the accommodation unit to move such that the accommodation unit is placed between the sucking member and the objective lens.

4. The cell sorting apparatus according to claim 2, further comprising a fixing mechanism configured to fix a biological culture chip to the support mechanism, and the biological culture chip is configured as the to-be-sorted member for accommodating the to-be-sorted cells.

5. The cell sorting apparatus according to claim 4, wherein the fixing mechanism comprises a chip fixing assembly configured to be placed on the support assembly and a support limiting assembly arranged on the support assembly for fixing the chip accommodation member onto the support assembly, wherein the chip fixing assembly comprises a chip accommodation member configured to accommodate the biological culture chip.

6. The cell sorting apparatus according to claim 1, wherein the imaging mechanism comprises an imaging lighting unit connected to the picking mechanism, an optical path assembly, and a detection assembly, wherein the imaging lighting unit is configured to provide lighting for the to-be-sorted member and the optical path assembly, the to-be-sorted member is arranged between the imaging lighting unit and the optical path assembly, the optical path assembly is configured to image the to-be-sorted cells and transfer the image information of the to-be-sorted cells to the detection assembly, and the detection assembly connected to the display mechanism is configured to obtain the image information of the to-be-sorted cells acquired by the optical path assembly.

7. The cell sorting apparatus according to claim 6, wherein the imaging mechanism further comprises a fluorescent assembly arranged on a same side of the to-be-sorted cells as the optical path assembly, wherein the fluorescent assembly comprises an excitation light source and at least one filter unit configured to filter excitation light emitted by the excitation light source, and wherein the light emitted by the excitation light source enables the to-be-sorted cells to emit fluorescence through the filtering unit.

8. The cell sorting apparatus according to claim 1, wherein the picking mechanism further comprises a mounting assembly, a picking movement assembly, and a recovery assembly that are mounted on the body, the mounting assembly configured to mount the sucking member, the picking movement assembly configured to drive the sucking member to move, to cause the sucking member to be close to or away from the support mechanism, picking movement assembly and the recovery assembly connected to the sucking member enables the sucking member to suck the cell meeting the preset requirement.

9. The cell sorting apparatus according to claim 8, wherein the picking mechanism further comprises a clamping unit configured to be mounted on the mounting assembly, the clamping unit configured to clamp and release the sucking member, and the clamping unit comprising at least one clamping member movable between a clamping position and a release position wherein the clamping member is configured to clamp at least part of the sucking member in the clamping position and release the sucking member in the release position.

10. The cell sorting apparatus according to claim 9, wherein the clamping unit further comprises a first clamping driving member configured to drive the clamping member to move between the clamping position and the release position and a clamping body configured to mount the first clamping driving member and the clamping member.

11. The cell sorting apparatus according to claim 10, wherein the clamping member is configured as an elastic member located between the first clamping driving member and the clamping body, and the elastic member is configured to change elastic deformation of the elastic member in a radial direction by adjusting a displacement between the first clamping driving member and the clamping body realizing clamping or releasing the sucking member.

12. The cell sorting apparatus according to claim 9, wherein the mounting assembly comprises an accommodation member configured to accommodate the clamping unit and at least one fixing member supported by the accommodation member, and wherein the fixing member is configured to fix the clamping unit onto the accommodation member.

13. The cell sorting apparatus according to claim 8, wherein the picking mechanism further comprises a picking lighting unit connected to the mounting assembly and located at an end of the sucking member away from the first support member, and light emitted by the picking lighting unit is passable through the sucking member.

14. The cell sorting apparatus according to claim 13, wherein the picking lighting unit comprises a picking lighting source extending in an axial direction of the sucking member and configured at an end of the sucking member provided with a hollow pipe, and light emitted by the picking lighting source is passable through the hollow pipe of the sucking member to form a sucking member projection.

15. The cell sorting apparatus according to claim 1, further comprising a cleaning assembly including a cleaning solution unit configured to clean the sucking member and a driving unit connected to the sucking member, wherein the sucking member is provided with a hollow pipe, and the cleaning solution unit comprises a first cleaning liquid and a second cleaning liquid different from the first cleaning liquid, and wherein the driving unit is configured to suck the first cleaning liquid into the hollow pipe for cleaning the sucking member preset times and suck the second cleaning liquid into the hollow pipe for cleaning the second preset times of the sucking member.
